# EUROPEAN PATENT APPLICATION

(11) **EP 1 992 272 A1**
(43) Date of publication of application: **19.11.2008**
(21) Application number: 08009099.6
(22) Date of filing: 16.05.2008
(51) Int. Cl.: A61B 1/005

(54) **Motor-driven bending endoscope**

(30) Priority: 17.05.2007 JP 2007131861
(71) Applicant: Olympus Medical Systems Corp., Tokyo 151-0072 (JP)
(72) Inventor: Adachi, Harutaka, Hachioji-shi Tokyo 192-8512 (JP); Sawai, Takashi, Hachioji-shi Tokyo 192-8512 (JP); Amano, Shoichi, Hachioji-shi Tokyo 192-8512 (JP); Itou, Seigo, Hachioji-shi Tokyo 192-8512 (JP); Ogawa, Atsushi, Hachioji-shi Tokyo 192-8512 (JP); Koitabashi, Masanobu, Hachioji-shi Tokyo 192-8512 (JP)
(74) Representative: von Hellfeld, Axel

(57) **Abstract**

A motor-driven bending endoscope includes a removal restriction mechanism (72) which restricts removal of a trackball (45) from a trackball mounting portion (70), and an operation section engaging portion (71) which detachably engages an attachment/detachment portion (63) with an operation section attachment portion (30) at a time of coupling between the operation section attachment portion (30) and the attachment/detachment portion (63) when an operation section unit (12) is attached to the operation section attachment portion (30). The operation section engaging portion (71) and the removal restriction mechanism (72) are interlockingly driven by an interlock drive mechanism (73). Thereby, there is provided a motor-driven bending endoscope which can reduce fatigue of the right hand at a time of performing a twisting operation of an insertion section, improve the recognizability of the up-and-down direction at a time of inserting the insertion section, and enhance the operational work efficiency.

## Description

The present invention relates to a motor-driven bending endoscope including an operation section which employs a trackball as an operation input device for operating a motor-driven bending section.

Conventionally, motor-driven bending endoscope apparatuses have been developed. In these apparatus, a bending section, which is provided in an insertion section of an endoscope, is bend-operated by a driving force of an electric motor. Jpn. Pat. Appln. KOKAI Publication No. 2003-275168 (patent document 1) discloses an example of the motor-driven bending endoscope apparatus. This apparatus includes an operation section which uses a trackball as an operation input device for operating a bending section.

Jpn. Pat. Appln. KOKAI Publication No. 2006-288751 (patent document 2) discloses another example of the motor-driven bending endoscope apparatus. In this apparatus, a driving unit is detachably coupled to a proximal end portion of an insertion section. A driving motor, which drives a bending section by motor driving, is assembled in the driving unit. Further, in this apparatus, a remote-control type operation section is provided. This operation section is disposed separately from the insertion section.

As regards the apparatus of patent document 1, patent document 1 shows no specific structure for rotatably engaging the trackball in the body of the operation section. Thus, the structure for engagement of the trackball is unclear. In the case where the trackball is rotatably engaged in the body of the operation section, the body of the operation section needs to be provided with engagement means of the trackball. In addition, when a work for attaching/detaching the trackball to/from the body of the operation section, it is necessary to perform a work for operating the engagement means of the trackball.

In the case where the remote-control type operation section is provided as in the apparatus of patent document 2, the insertion section and the operation section are separated. In general, when the endoscope is used, the insertion section is grasped, for example, by the right hand, and the operation section is grasped by the left hand. Thus, unlike the operation of the conventional endoscope, when an operation for twisting the insertion section i's performed, the operation of twisting the insertion section cannot be supported by the left hand that holds the operation section. As a result, when the operation of twisting the insertion section is performed, the insertion section is twisted by the right hand alone, and the fatigue of the right hand of the operator who operates the endoscope increases.

Moreover, in the case of the remote-control type operation section, since the insertion section and the operation section are separated, the insertion section is grasped by the right hand alone. Consequently, if the operation of inserting the insertion section is continuously performed, there is a tendency that the up-and-down direction of the insertion section is not understood by feeling. As a result, the work efficiency of the operation for inserting the insertion section may possibly deteriorate.

The present invention has been made in consideration of the above-described circumstances, and the object of the invention is to provide a motor-driven bending endoscope which can reduce fatigue of the right hand at a time of performing a twisting operation of an insertion section, improve the recognizability of the up-and-down direction at a time of inserting the insertion section, and enhance the operational work efficiency.

According to an aspect of the present invention, there is provided a motor-driven bending endoscope comprising: an operation section attachment portion which is provided on a proximal end portion of an insertion section including a motor-driven bending section; and an operation section unit which is detachably attached to the operation section attachment portion and includes an operation input section which operates an endoscope, wherein the operation section unit comprises: a trackball mounting portion; a trackball which is rotatably and attachably/detachably mounted in the trackball mounting portion and is an instruction section for instructing an operation of the bending section; a removal restriction mechanism which restricts removal of the trackball, which is mounted in the trackball mounting portion, from the trackball mounting portion; an attachment/detachment portion which is detachably coupled to the operation section attachment portion when the operation section unit is attached to the operation section attachment portion; an operation section engaging portion which detachably engages the attachment/detachment portion with the operation section attachment portion when the operation section attachment portion and the attachment/detachment portion are coupled; and an interlock drive mechanism which is coupled to the operation section engaging portion and the removal restriction mechanism and interlockingly drives the operation section engaging portion and the removal restriction mechanism.

In the above-described structure, the operation section unit is detachably attached to the operation section attachment portion at the proximal end portion of the insertion section. Thereby, the fatigue of the right hand at a time of performing a twisting operation of the insertion section can be reduced, the recognizability of the up-and-down direction at a time of inserting the insertion section can be improved, and the operational work efficiency can be enhanced. Moreover, at the time of the work for attaching/detaching the operation section unit to/from the operation section attachment portion, the operation section engaging portion and the removal restriction mechanism are interlockingly driven by operating the interlock drive mechanism. When the operation section unit is attached to the operation section attachment portion, the attachment/detachment portion of the operation section unit is detachably engaged with the operation section attachment portion by the operation section engaging portion, and the removal restriction mechanism restricts removal of the trackball which is mounted in the trackball mounting portion. Therefore, the operation of preventing removal of the operation section unit, which is removable from the operation section attachment portion, and the operation of preventing removal of the trackball, which is removable from the operation section unit, can be performed at the same time and the operation is facilitated.

Preferably, the proximal end portion of the insertion section includes a coupling member which is detachably coupled to a power unit which includes a driving force source for supplying a driving force to the insertion section, and the operation section unit instructs an operation of the driving force source.

In the above-described structure, the coupling member at the proximal end portion of the insertion section is detachably coupled to the power unit, and the operation section unit instructs an operation of the driving force source of the power unit. Thereby, the driving force source for supplying a driving force to the insertion section is controlled.

Preferably, the operation section attachment portion includes an engaging portion for detachably engaging the attachment/detachment portion of the operation section unit, and a guide portion for guiding the attachment/detachment portion of the operation section unit to a position of the engaging portion, the guide portion includes guide rails which guide the operation section unit in two different directions, a first guide direction of the guide rails is different from a direction of attachment/detachment of the proximal end portion of the insertion section to/from the power unit, and a second guide direction of the guide rails is set to be different from the first guide direction.

In the above-described structure, at the time of the work for attaching/detaching the operation section unit to/from the operation section attachment portion, the attachment/detachment portion of the operation section unit is guided to the position of the engaging portion along the guide portion. At this time, the operation section unit is guided by guide rails of the guide portion in the two different directions, that is, in a first guide direction that is set to be different from a direction of attachment/detachment of the proximal end portion of the insertion section to/from the power unit, and a second guide direction that is set to be different from the first guide direction.

Preferably, the second guide direction is set to be such a direction as to have resistance against a direction of twisting of the insertion section about a center axis of the insertion section.

In the above-described structure, when the operation section unit is attached to the operation section attachment portion, the second guide direction of the operation section unit is set to be such a direction as to have resistance against a direction of twist of the axial rotation about a center axis of the insertion section. Thereby, the operation section is offset from the rotational shaft, and the twisting operation is facilitated.

Preferably, the operation section unit is provided with the trackball mounting portion on a front surface side of an operation section unit body and is provided with the attachment/detachment portion on a back surface side of the operation section unit body, the trackball mounting portion is formed on the operation section unit and includes a recess portion for attachment of the trackball, and the removal restriction mechanism includes a stopper pin which is advancible/retreatable in the recess portion in accordance with an operation of the interlock drive mechanism.

In the above-described structure, when the trackball is attached to the recess portion of the trackball mounting portion on the front surface side of the operation section unit body, the stopper pin of the removal restriction means is advanced/retreated in the recess portion in accordance with the operation of the interlock drive mechanism. Thereby, the trackball is engaged with the stopper pin, and removal of the trackball from the recess portion of the trackball mounting portion is restricted.

Preferably, in the operation section unit a first center axis which is a center of grasping at a time of grasping is eccentric to a second center axis which is a center axis of the insertion section.

In the above-described structure, the first center axis which is the center of grasping at a time of grasping the operation section unit is positioned to be eccentric to the second center axis which is the center axis of the insertion section. Thereby, when the operation section unit is attached to the operation section attachment portion, the operation section is offset from the rotational shaft, and the twisting operation is facilitated.

Preferably, the operation input section includes a bending operation input device, an air-feed/water-feed switch, a suction switch and a scope switch.

In the above-described structure, the operation input section of the operation section unit is configured to operate a bending operation input device, an air-feed/water-feed switch, a suction switch and a scope switch.

Preferably, the operation section attachment portion includes first guide rails which guide the operation section unit in the first guide direction, and second guide rails which guide the operation section unit in the second guide direction, and the operation section engaging portion is operated at a position where the attachment/detachment portion of the operation section unit is moved to a terminal end position of the second guide rails.

In the above-described structure, when the operation section unit is attached to the operation section attachment portion, the operation section unit is guided in the first guide direction by the first guide rails of the operation section attachment portion, and then the operation section unit is guided in the second guide direction by the second guide rails. At this time, the operation section engaging portion is operated at a position where the operation section unit is moved to the terminal end position of the second guide rails.

Preferably, the interlock drive mechanism includes a first operation portion which operates the operation section engaging portion, a second operation portion which operates the removal restriction mechanism, and a rotational lever which rotates about a rotational shaft, the rotational lever includes a lever operation portion at one end side, and includes the first operation portion at the other end side, and the first operation portion is operated at a terminal end position of a range of rotation of the rotational lever, and the second operation portion is operated at an intermediate position of the range of rotation of the rotational lever.

In the above-described structure, when the operation section unit is attached to the operation section attachment portion, the lever operation portion at one end side of the rotational lever is operated to rotate the rotational lever about the rotational shaft. Thereby, the operation section engaging means is operated by the first operation portion, and the removal restriction means is operated by the second operation portion. At this time, the first operation portion is operated at the terminal end position of the range of rotational movement of the rotational lever, and the second operation portion is operated at the intermediate position of the range of rotational movement. Thereby, when the operation section unit is detached from the operation section attachment portion, the timing at which the operation section unit is detached from the operation section attachment portion is made different from the timing at which the trackball is removed from the operation section unit in accordance with the angle of operation of the lever. When the lever is rotated over 45°, the operation section unit is detached. When the lever is rotated over 90°, the trackball is removed.

Preferably, the operation section engaging portion includes a fixing portion which is projectingly provided on the operation section attachment portion, and the rotational lever is moved to the terminal end position of the range of rotation when the attachment/detachment portion of the operation section unit is moved to a terminal end position of the second guide rails, thereby engaging the first operation portion with the fixing portion and restricting movement of the operation section unit along the second guide rails.

In the above-described structure, when the operation section unit is attached to the operation section attachment portion, the rotational lever is moved to the terminal end position of the range of rotational movement in the state that the attachment/detachment portion of the operation section unit is moved to the terminal end position of the second guide portion, thereby engaging the first operation with the engaging portion of the operation section attachment portion and restricting movement of the operation section unit along the second guide portion.

According to another aspect of the present invention, there is provided a motor-driven bending endoscope comprising: an operation section attachment portion which is provided on a proximal end portion of an insertion section including a motor-driven bending section; and an operation section unit which is detachably attached to the operation section attachment portion and includes an operation input section which operates an endoscope, wherein the operation section unit comprises: a trackball mounting portion; a trackball which is rotatably and attachably/detachably mounted in the trackball mounting portion and is an instruction section for instructing an operation of the bending section; removal restriction means for restricting removal of the trackball, which is mounted in the trackball mounting portion, from the trackball mounting portion; an attachment/detachment portion which is detachably coupled to the operation section attachment portion when the operation section unit is attached to the operation section attachment portion; operation section engaging means for detachably engaging the attachment/detachment portion with the operation section attachment portion when the operation section attachment portion and the attachment/detachment portion are coupled; and interlock drive means which is coupled to the operation section engaging means and the removal restriction means and interlockingly drives the operation section engaging means and the removal restriction means.

According to the present invention, the fatigue of the right hand at a time of performing a twisting operation of the insertion section can be reduced, the recognizability of the up-and-down direction at a time of inserting the insertion section can be improved, and the work efficiency of the operation of inserting the insertion section can be enhanced.

The invention can be more fully understood from the following detailed description when taken in conjunction with the accompanying drawings, in which:
FIG. 1 is a perspective view that schematically shows the structure of the entire system of a medical apparatus according to a first embodiment of the present invention;
FIG. 2 is a block diagram that schematically shows the medical apparatus according to the first embodiment;
FIG. 3 is a side view that shows the state of connection between a proximal end portion of an insertion section of a motor-driven bending endoscope according to the first embodiment and a power unit, with a part of the power unit being shown in cross section;
FIG. 4A is a side view showing the proximal end portion of the insertion section of the motor-driven bending endoscope according to the first embodiment;
FIG. 4B is a side view showing an operation section unit of the motor-driven bending endoscope according to the first embodiment;
FIG. 4C is a longitudinal cross-sectional view of the power unit of the motor-driven bending endoscope according to the first embodiment;
FIG. 4D is a side view showing an endoscope holding section of an endoscope arm which holds the motor-driven bending endoscope according to the first embodiment;
FIG. 4E is a side view showing a conduit connector of the motor-driven bending endoscope according to the first embodiment;
FIG. 5 is a perspective view that shows the state before the proximal end portion of the insertion section of the motor-driven bending endoscope of the medical apparatus according to the first embodiment and the power unit are connected;
FIG. 6 schematically shows the structure of the insertion section of the motor-driven bending endoscope according to the first embodiment;
FIG. 7 is a side view of an insertion section connector of the motor-driven bending endoscope according to the first embodiment;
FIG. 8 is a front view of the insertion section connector of the motor-driven bending endoscope according to the first embodiment;
FIG. 9A is a plan view showing the state in which the power unit is attached to a C-shaped ring of the endoscope holding section which is provided on the endoscope arm of the medical apparatus according to the first embodiment;
FIG. 9B is a plan view showing the state in which a movable portion of the C-shaped ring is moved according to the first embodiment;
FIG. 10 is a perspective view showing the state in which the proximal end portion of the insertion section of the motor-driven bending endoscope according to the first embodiment, the power unit and the operation section unit are separated;
FIG. 11 is a perspective view that shows a region of an attachment/detachment part of the operation section unit in the state in which the operation section unit of the motor-driven bending endoscope according to the first embodiment is detached from an operation section attachment portion;
FIG. 12 is a perspective view that shows a region of an operation section attachment portion of the insertion section connector and a region of the attachment/detachment portion of the operation section unit in the state in which the operation section unit of the motor-driven bending endoscope according to the first embodiment is detached from the operation section attachment portion;
FIG. 13 is a side view showing the state in which the operation section unit of the motor-driven bending endoscope according to the first embodiment is detached from the operation section attachment portion;
FIG. 14 is a plan view showing the attachment/detachment portion of the operation section unit of the motor-driven bending endoscope according to the first embodiment;
FIG. 15 is a perspective view showing slide ribs of the operation section unit of the motor-driven bending endoscope according to the first embodiment;
FIG. 16 is a perspective view showing first and second guide rail structure members and a fixing rib of the operation section attachment portion of the motor-driven bending endoscope according to the first embodiment;
FIG. 17 is a dissolved perspective view showing the structure of interlock driving means between operation section engaging means and removal restriction means of the motor-driven bending endoscope according to the first embodiment;
FIG. 18 is a perspective view showing a coupling structure between an attachment/detachment operation lever and interlock drive means of the motor-driven bending endoscope according to the first embodiment;
FIG. 19 is a perspective view showing a rotation state of the attachment/detachment operation lever of the motor-driven bending endoscope according to the first embodiment;
FIG. 20 is a perspective view showing the state in which a bracket of the interlock drive means of the motor-driven bending endoscope according to the first embodiment is assembled;
FIG. 21A is a perspective view showing the state in which a stopper pin of a trackball of the motor-driven bending endoscope of the first embodiment is moved in a trackball engagement release direction;
FIG. 21B is a side view of the structure shown in FIG. 21A;
FIG. 22A is a perspective view showing the state in which the stopper pin of the trackball of the motor-driven bending endoscope of the first embodiment is moved in a trackball engagement direction;
FIG. 22B is a side view of the structure shown in FIG. 22A;
FIG. 23 is a perspective view showing the entirety of the interlock drive means of the motor-driven bending endoscope according to the first embodiment;
FIG. 24 is a plan view showing the state in which an engaging pin provided at a distal end portion of a plate spring is engaged in an engaging groove of a rotational shaft of the attachment/detachment operation lever of the motor-driven bending endoscope according to the first embodiment;
FIG. 25 is a perspective view showing the state in which the attachment/detachment operation lever is held in an unlocked position in a state in which the operation section unit of the motor-driven bending endoscope according to the first embodiment is attached to the operation section attachment portion;
FIG. 26 is a plan view showing the state in which the attachment/detachment operation lever is held in the unlocked position in the state in which the operation section unit of the motor-driven bending endoscope according to the first embodiment is attached to the operation section attachment portion;
FIG. 27 is a longitudinal cross-sectional view showing the state in which the attachment/detachment operation lever is held in the unlocked position in the state in which the operation section unit of the motor-driven bending endoscope according to the first embodiment is attached to the operation section attachment portion;
FIG. 28 is a longitudinal cross-sectional view showing the state in which the attachment/detachment operation lever is held in the unlocked position in the state in which the operation section unit of the motor-driven bending endoscope according to the first embodiment is attached to the operation section attachment portion;
FIG. 29 is a perspective view showing the state in which the attachment/detachment operation lever is rotated to an intermediate lock position in the state in which the operation section unit of the motor-driven bending endoscope according to the first embodiment is attached to the operation section attachment portion;
FIG. 30 is a plan view showing the state in which the attachment/detachment operation lever is rotated to the intermediate lock position in the state in which the operation section unit of the motor-driven bending endoscope according to the first embodiment is attached to the operation section attachment portion;
FIG. 31 is a longitudinal cross-sectional view showing the state in which the attachment/detachment operation lever is rotated to the intermediate lock position in the state in which the operation section unit of the motor-driven bending endoscope according to the first embodiment is attached to the operation section attachment portion;
FIG. 32 is a perspective view showing the state in which the attachment/detachment operation lever is rotated to a lock position in the state in which the operation section unit of the motor-driven bending endoscope according to the first embodiment is attached to the operation section attachment portion;
FIG. 33 is a plan view showing the state in which the attachment/detachment operation lever is rotated to the lock position in the state in which the operation section unit of the motor-driven bending endoscope according to the first embodiment is attached to the operation section attachment portion;
FIG. 34 is a longitudinal cross-sectional view showing the state in which the attachment/detachment operation lever is rotated to the intermediate lock position in the state in which the operation section unit of the motor-driven bending endoscope according to the first embodiment is attached to the operation section attachment portion;
FIG. 35 is a longitudinal cross-sectional view of a main part, showing the state in which the attachment/detachment operation lever is rotated to the lock position in the state in which the operation section unit of the motor-driven bending endoscope according to the first embodiment is attached to the operation section attachment portion;
FIG. 36 is a longitudinal cross-sectional view showing the state in which the attachment/detachment operation lever is rotated to the lock position in the state in which the operation section unit of the motor-driven bending endoscope according to the first embodiment is attached to the operation section attachment portion;
FIG. 37 is a transverse cross-sectional view showing the state in which the trackball is mounted in a recess portion of a trackball mounting section in the operation section unit of the motor-driven bending endoscope according to the first embodiment; and
FIG. 38 is a side view showing a clearance between the fixing rib and the attachment/detachment portion of the operation section unit in the state in which the operation section unit of the motor-driven bending endoscope according to the first embodiment is attached to the operation section attachment portion.

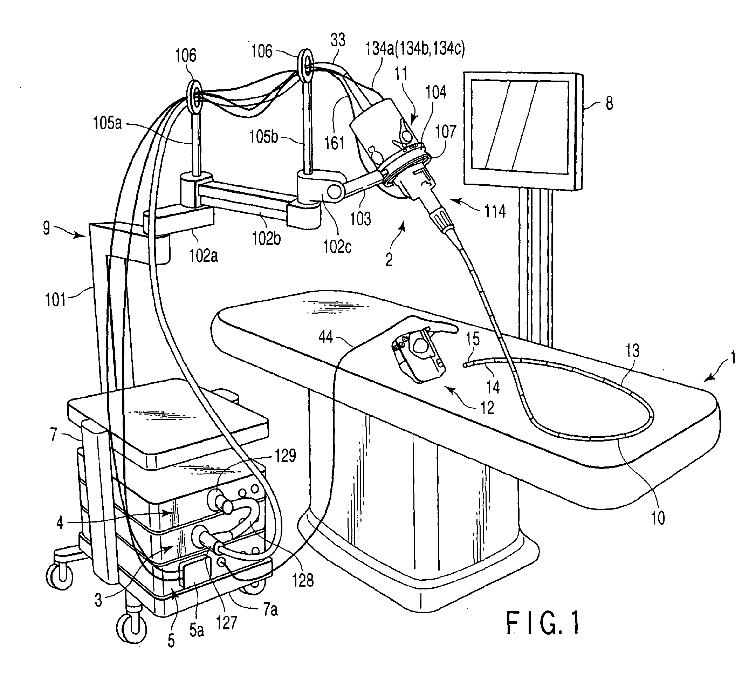

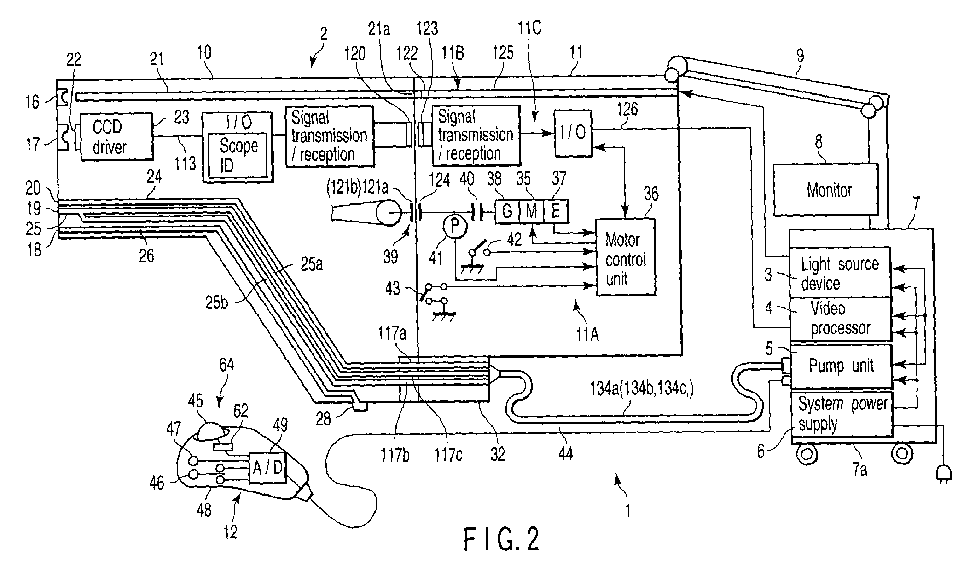

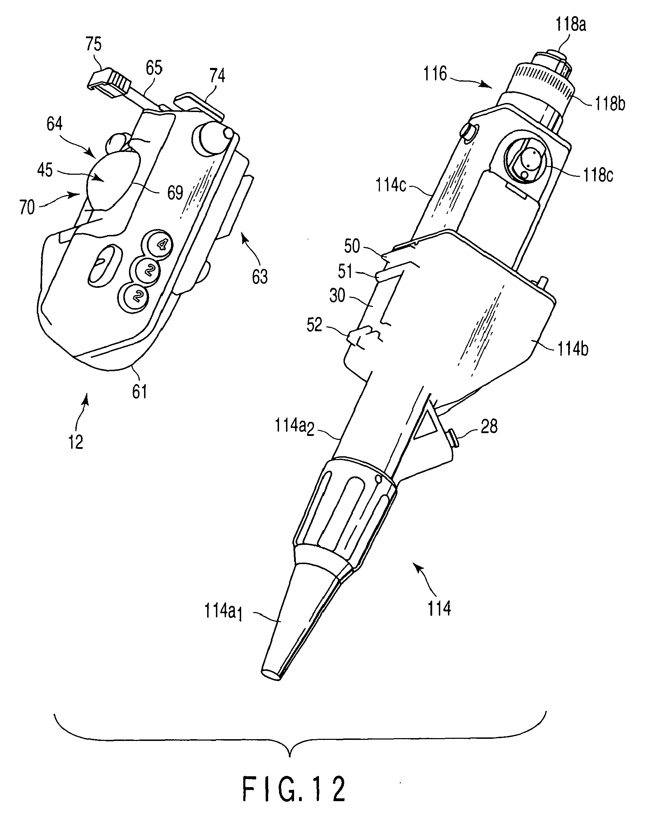

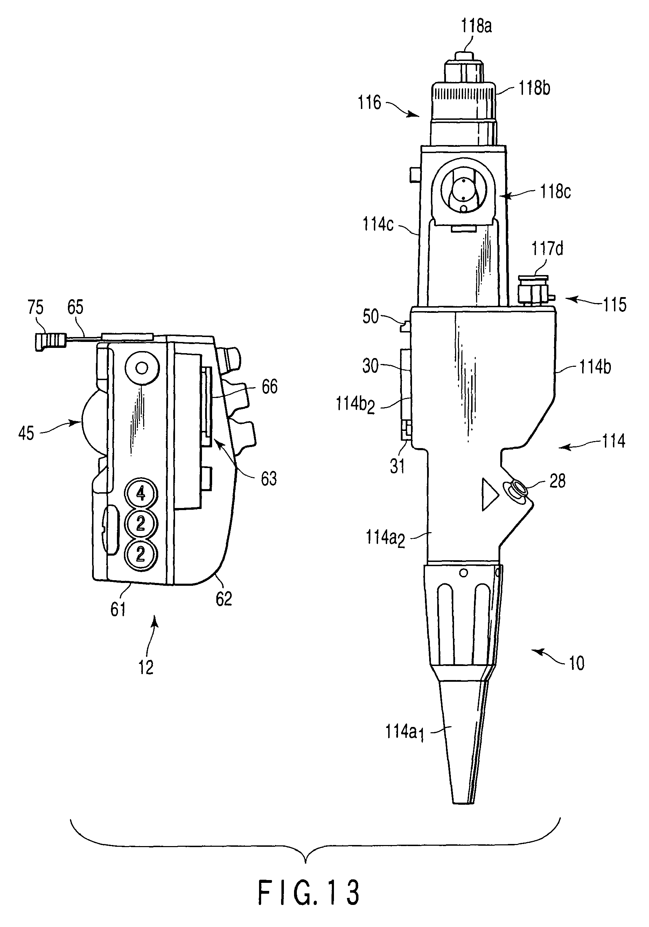

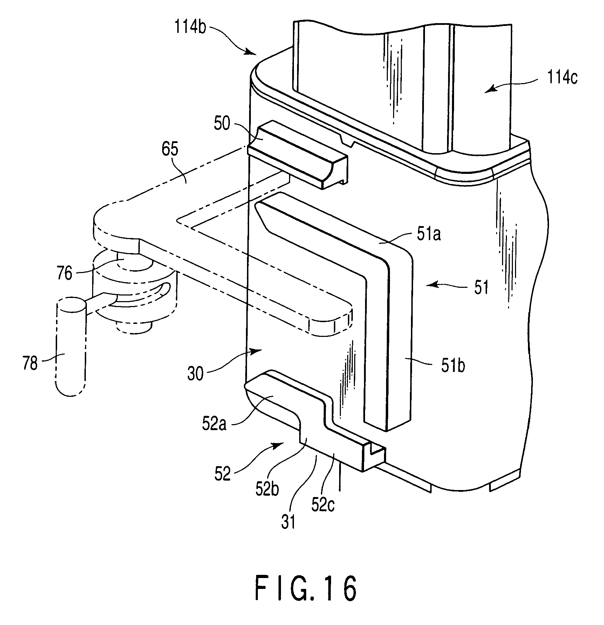

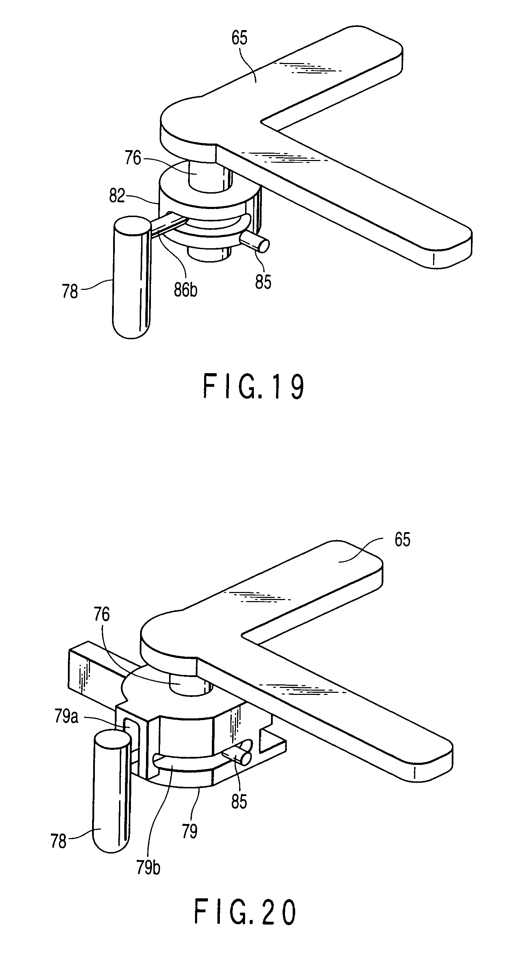

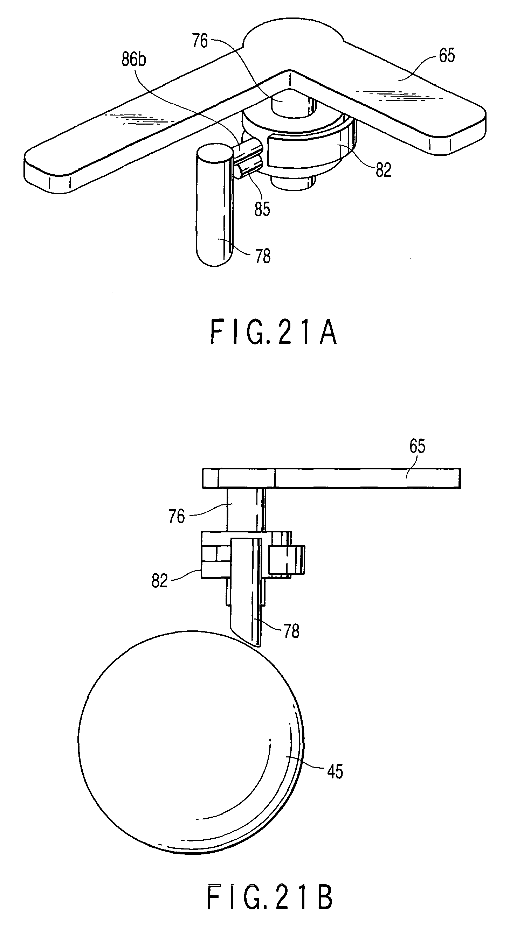

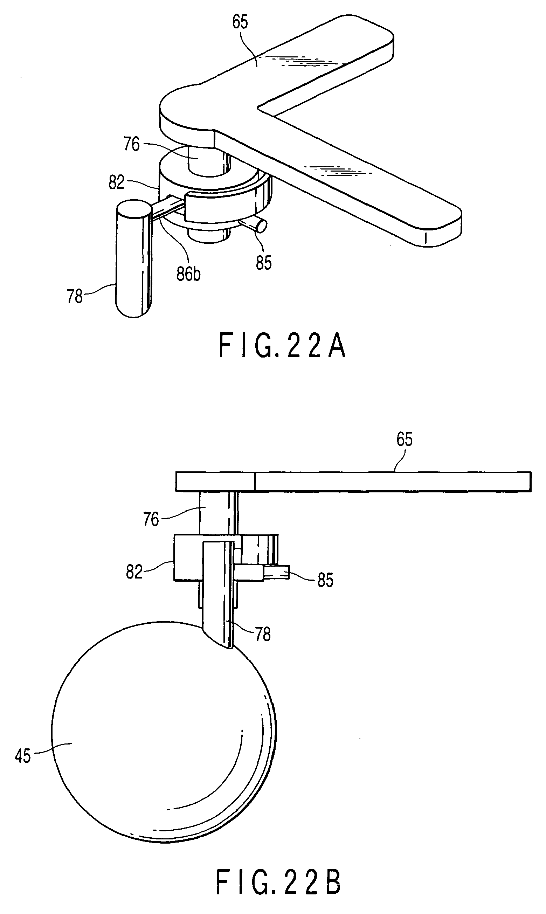

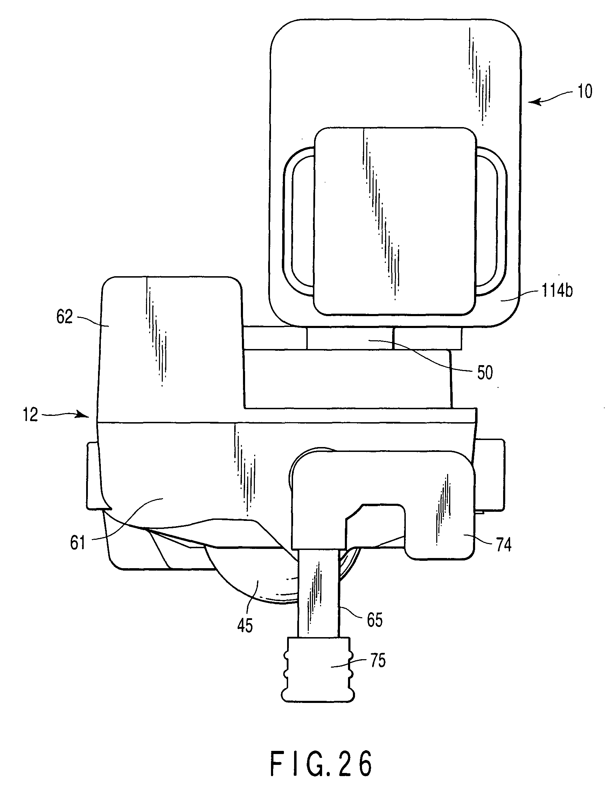

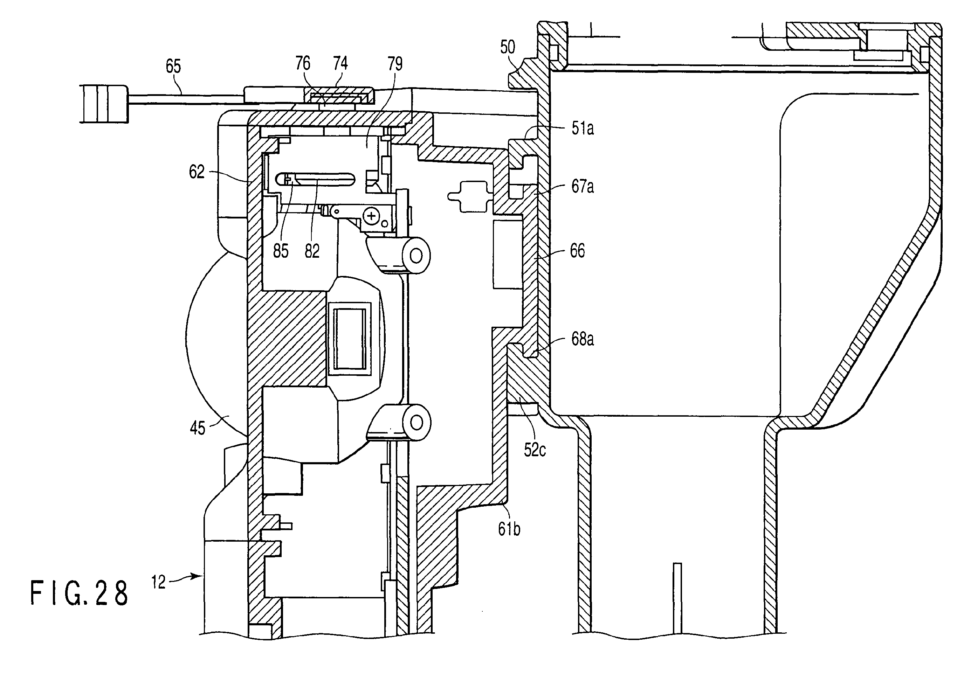

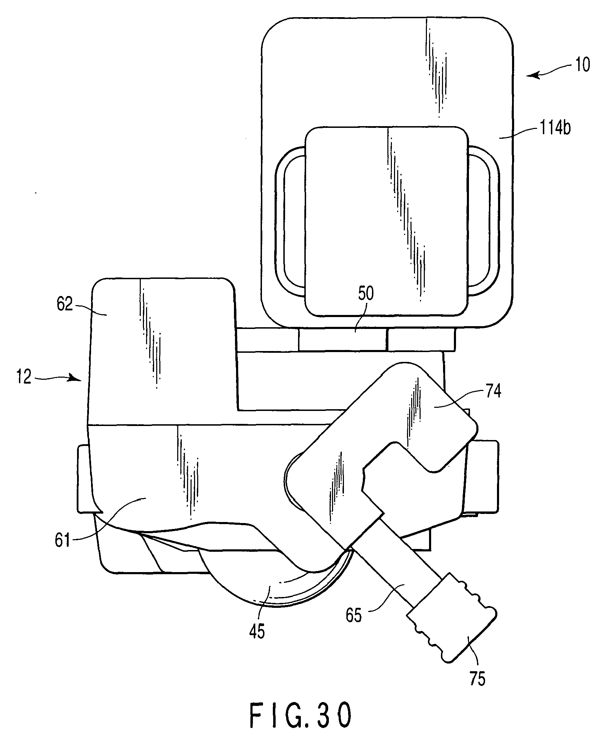

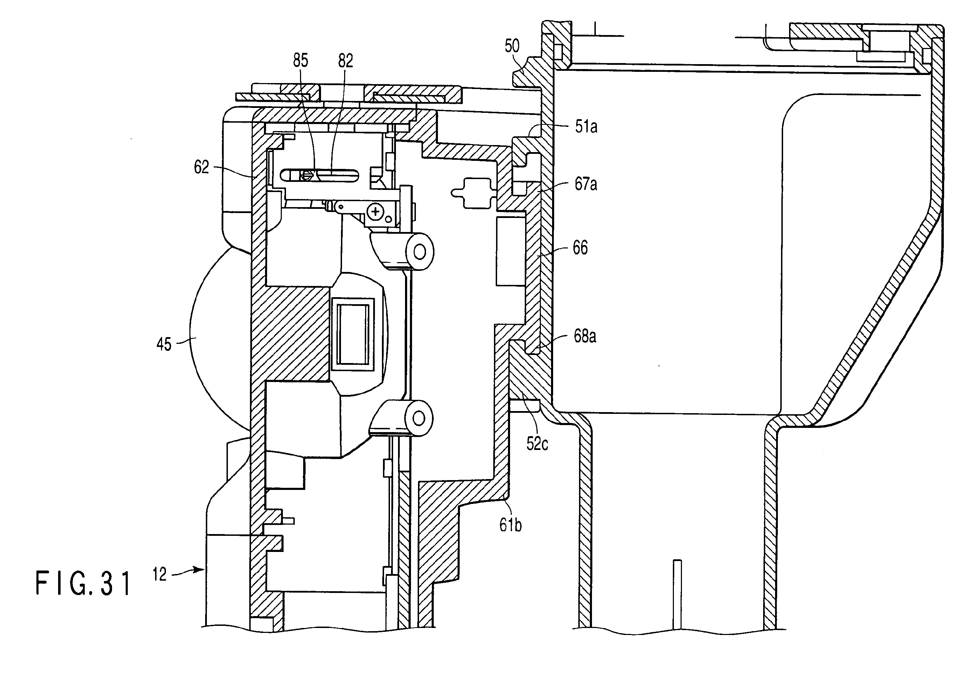

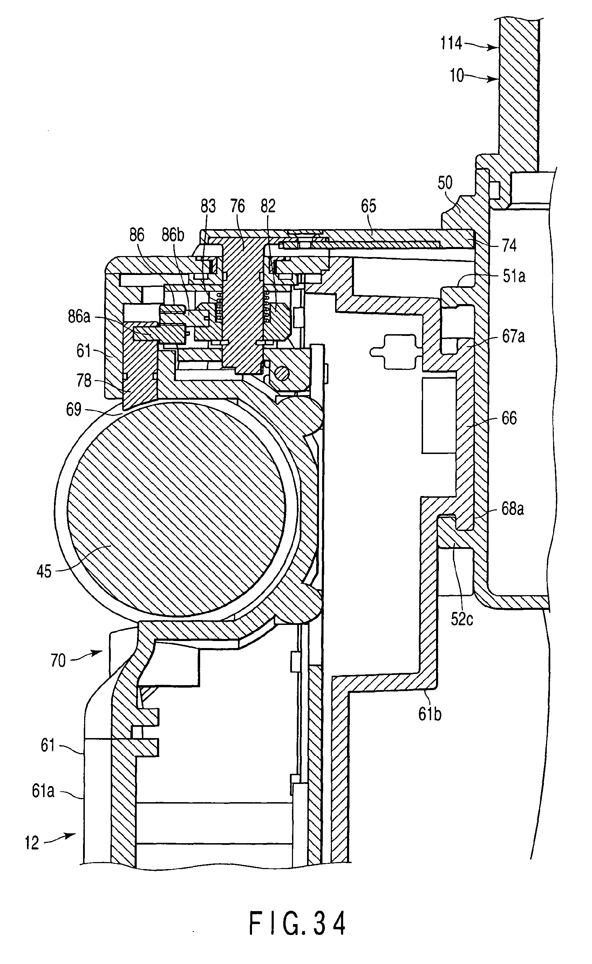

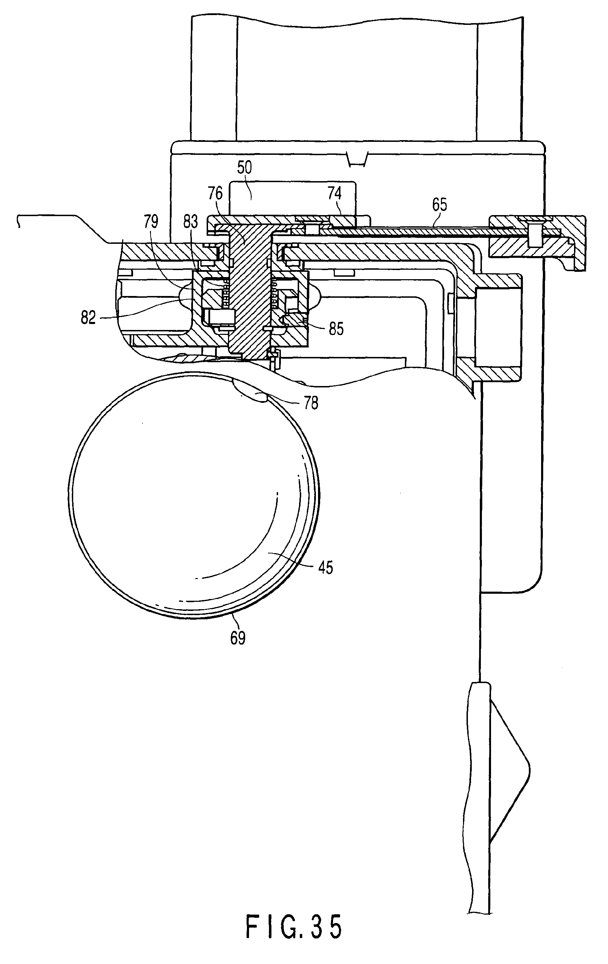

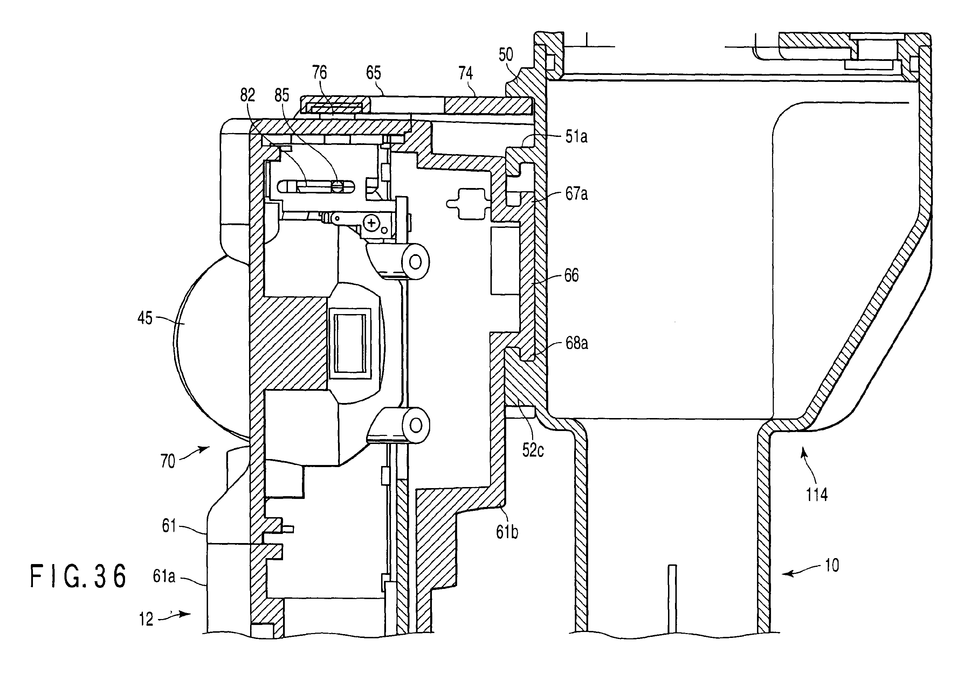

A first embodiment of the present invention will now be described with reference to from FIG. 1 to FIG. 38. FIG. 1 schematically shows the structure of the entire system of a medical apparatus. In the medical apparatus, a cart 7, on which the system of a motor-driven bending endoscope 2 is mounted, is disposed near a patient bed 1. A motor-driven bending endoscope apparatus, which is the medical apparatus, mainly comprises the motor-driven bending endoscope 2, a light source device 3, a video processor 4, a pump unit 5 and a system power supply 6 (see FIG. 2).

As shown in FIG. 2, the cart 7 includes a carrier 7a having, for example, casters for free movement on the floor. The light source device 3, video processor 4, pump unit 5 and system power supply 6 are mounted on the carrier 7a in a stacked fashion. The system of the motor-driven bending endoscope 2 includes a monitor 8 which receives a video signal from the video processor 4 and displays a predetermined endoscopic image. The monitor 8 is mounted in the state in which the monitor 8 is erectingly provided beside the patient bed 1.

Further, the cart 7 includes an endoscope arm 9 which is composed of, for instance, a plurality of movable arm portions which movably support the motor-driven bending endoscope 2. The endoscope arm 9 includes a substantially L-shaped support arm 101 which is erectly provided on the cart 7, a plurality (three (first to third) in the present embodiment) of horizontal movement arms 102a, 102b and 102c, and one vertical movement arm 103. One end portion of the first horizontal movement arm 102a is coupled to an upper end portion of the support arm 101 so as to be rotatable about a rotational shaft which is vertically extendingly provided. One end portion of the second horizontal movement arm 102b is coupled to the other end portion of the first horizontal movement arm 102a so as to be rotatable about a rotational shaft which is vertically extendingly provided. One end portion of the third horizontal movement arm 102c is coupled to the other end portion of the second horizontal movement arm 102b so as to be rotatable about a rotational shaft which is vertically extendingly provided. A proximal end portion of the vertical movement arm 103 is coupled to the other end portion of the third horizontal movement arm 102c so as to be rotatable about a rotational shaft which is horizontally extendingly provided. Furthermore, an endoscope holding section 104 is disposed at a distal end portion of the vertical movement arm 103.

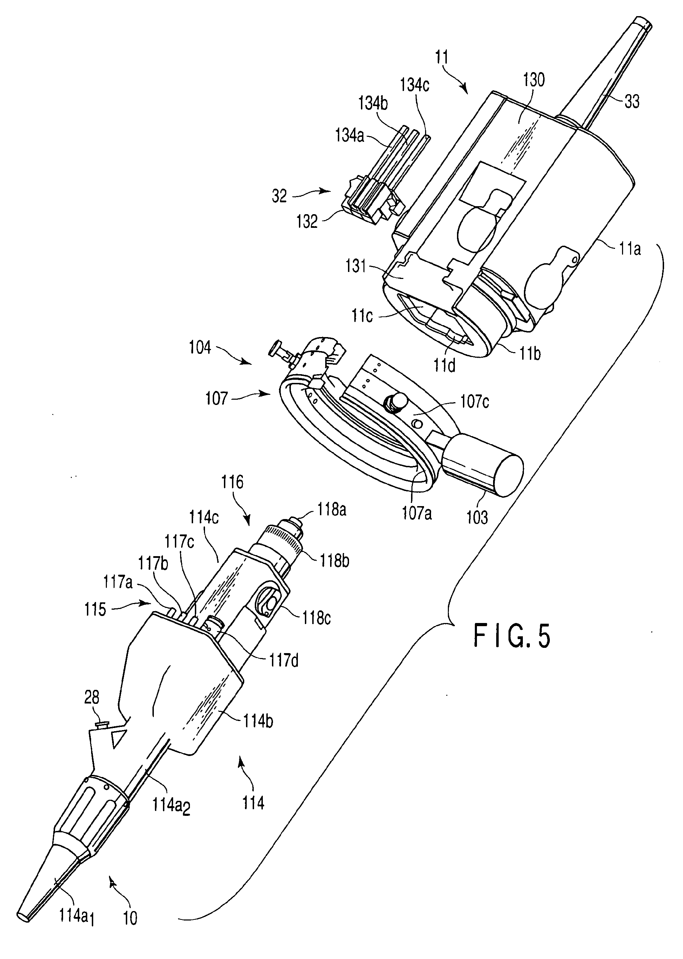

As shown in FIG. 5, the endoscope holding section 104 of the endoscope arm 9 includes a C-shaped ring 107 having a substantially C-shaped form. The C-shaped ring 107 includes a substantially C-shaped inner ring portion 107a and a substantially C-shaped outer ring portion 107b. The inner ring portion 107a is coupled so as to be rotatable along the outer ring portion 107b, and is so supported that the inner ring portion 107a can be fixed at an arbitrary rotational position.

In addition, a first support rod 105a is vertically erectly coupled to a coupling part between the first horizontal movement arm 102a and the second horizontal movement arm 102b, and a second support rod 105b is vertically erectly coupled to a coupling part between the second horizontal movement arm 102b and the third horizontal movement arm 102c. Ring-shaped bundle retainers 106 are fixed to upper end portions of the first support rod 105a and second support rod 105b, respectively.

The motor-driven bending endoscope 2 mainly comprises an elongated insertion section 10 which is inserted in a body cavity, a power unit 11 which is detachably coupled to a proximal end portion of the insertion section 10, a conduit connector 32 which is detachably coupled to the power unit 11, and an operation section unit 12.

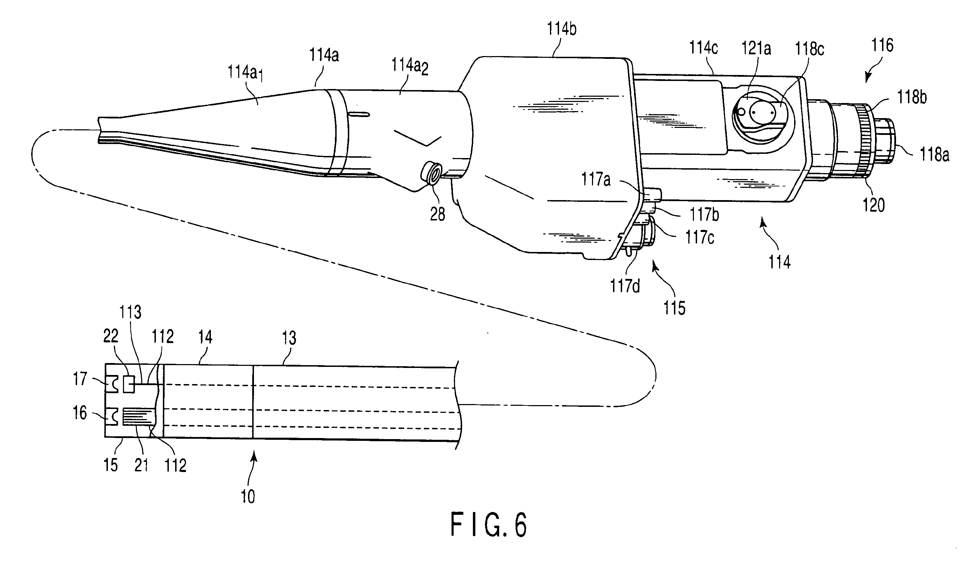

As shown in FIG. 6, the insertion section 10 includes an elongated flexible tube section 13, a bending section 14 having a proximal end portion connected to a distal end of the flexible tube section 13, and a distal-end rigid section 15 having a proximal end portion connected to a distal end of the bending section 14. A distal end face of the distal-end rigid section 15 is provided with an illumination lens 16 of an illumination optical system, an observation lens 17 of an observation optical system, a distal-end opening portion 18 of a therapeutic device insertion channel shown in FIG. 2, an air-feed/water-feed nozzle 19, and a forward water-feed opening portion 20.

A distal end portion of a light guide 21 is disposed behind the illumination lens 16. The light guide 21 is formed of an optical fiber which guides illumination light. An image pick-up element, such as a CCD 22, and a CCD driver 23 are disposed behind the observation lens 17. The CCD 22 photoelectrically converts an image which is focused by the observation lens 17. The CCD driver 23 drives the CCD 22.

The bending section 14 of the motor-driven bending endoscope 2 is configured such that a plurality of substantially ring-shaped bend pieces are juxtaposed in the axial direction of the insertion section 10. The plural bend pieces are rotatably coupled via rotational pins such as rivets. Distal end sides of four wires for a bending operation are connected to the bending section 14. The four wires bend-operate the bending section 14, for example, in four directions, that is, upward, downward, leftward and rightward directions. Proximal end sides of the wires are extended toward the proximal end side of the insertion section 10. One or two of the wires, which receive a driving force from the power unit 11, are pulled and driven. Thereby, the bending section 14 can be bent from a normal straight state (non-bent state) in which the bending section 14 is straight and the bend angle is 0° to a bent shape in which the bending section 14 is bend-operated in a bent shape at an arbitrary angle in one of upward, downward, leftward and rightward directions.

The insertion section 10 contains first built-in components 111 and second built-in components 112. The first built-in components 111 are a plurality of built-in components which are detachably connected to an external device, for instance, fluid conduits. The second built-in components 112 are composed of transmission paths for transmitting a driving force, light and signals.

In the present embodiment, the first built-in components 111 comprise a forward water-feed conduit 24, an air-feed conduit 25a, a water-feed conduit 25b and a therapeutic device insertion conduit 26 which serves also as a suction conduit. The second built-in components 112 comprise, for instance, four wires (not shown) for a bending operation (for driving power transmission) for bend-operating the bending section 14 in four directions, i.e. upward, downward, leftward and rightward directions, a light guide 21 for light transmission, and an electric line 113 for signal transmission which is connected to the CCD driver 23.

A distal end portion of the forward water-feed conduit 24 is coupled to the forward water-feed opening portion 20. A distal end portion of the water-feed conduit 25b is connected to a distal end portion of the air-feed conduit 25a. An air-feed/water-feed conduit 25 is formed at a distal end side of the connection part between the water-feed conduit 25b and the air-feed conduit 25a. A distal end portion of the air-feed/water-feed conduit 25 is coupled to the air-feed/water-feed nozzle 19. A distal end portion of the therapeutic device insertion conduit 26 is coupled to the distal-end opening portion 18.

As shown in FIG. 6, a proximal end portion of the insertion section 10 is provided with an insertion section connector 114. The insertion section connector 114 has a greater diameter than the flexible tube section 13. The insertion section connector 114 includes three blocks which are disposed along the axial direction, namely, a first block 114a, a second block 114b and a third block 114c. The first block 114a includes a conical portion 114a1 which is positioned closest to the flexible tube section 13 side, and a circular cylindrical portion 114a2. The second block 114b is a largest block which is disposed at the central position. The third block 114c is a block which is disposed at a terminal part of the proximal end portion of the insertion section 10. The insertion section connector 114 is configured such that the part of the third block 114c is detachably inserted in the power unit 11 in the insertion direction of the insertion section 10 and is detachably coupled.

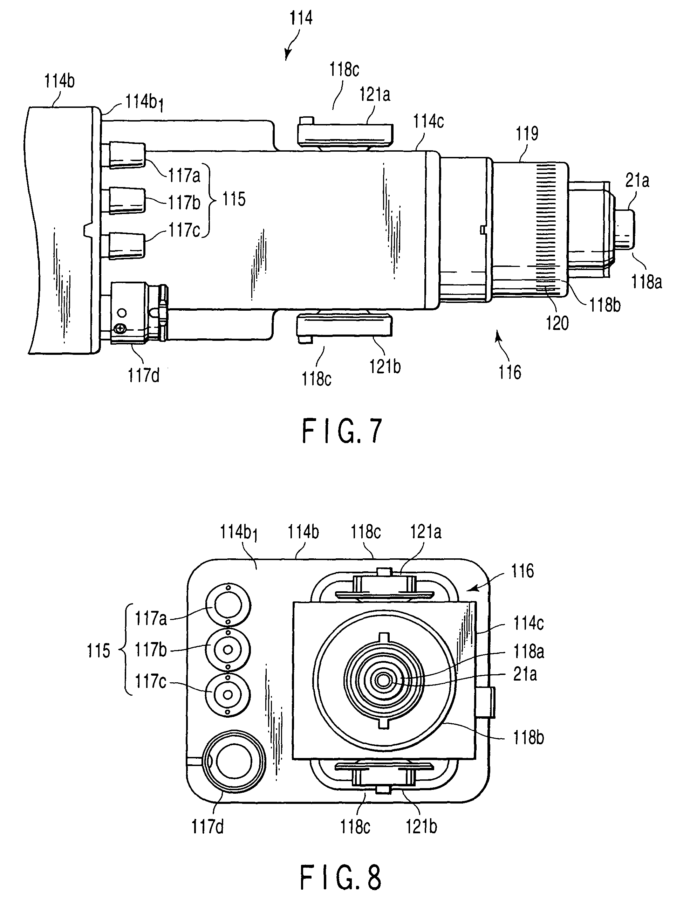

Furthermore, the insertion section connector 114 includes a first connector portion 115 and a second connector portion 116. The first connector portion 115 is provided with a forward water-feed mouthpiece 117a, a water-feed mouthpiece 117b, an air-feed mouthpiece 117c and a water leak detection mouthpiece 117d. As shown in FIG. 7, these mouthpieces from 117a to 117d are provided on an end face 114b1 of the second block 114b, which is located between the second block 114b and third block 114c of the insertion section connector 114, in such a manner that the mouthpieces from 117a to 117d project rearward. A proximal end portion of the forward water-feed conduit 24 is coupled to the forward water-feed mouthpiece 117a. Similarly, a proximal end portion of the water-feed conduit 25b is coupled to the water-feed mouthpiece 117b, and a proximal end portion of the air-feed conduit 25a is coupled to the air-feed mouthpiece 117c. The mouthpieces from 117a to 117c are composed of male mouthpieces of a rigid material such as a metal.

The second connector portion 116 is provided with an optical connection section 118a, an electrical connection section 118b and a driving force transmission section 118c for transmitting a driving force. A circular cylindrical member 119 is projectingly provided on a rear end portion of the third block 114c of the insertion section connector 114. The electrical connection section 118b is composed of electric contacts 120 which are juxtaposed on an outer peripheral surface of the circular cylindrical member 119.

A connection terminal portion 21a of the light guide 21 is disposed at an axial central part of the circular cylindrical member 119 at the rear end portion of the third block 114c. The optical connection section 118a is formed of the connection terminal portion 21a of the light guide 21. The driving force transmission section 118c is formed of a pair of coupling portions 121a and 121b which are disposed on upper and lower sides (in FIG. 7 and FIG. 8) of the third block 114c of the insertion section connector 114.

A therapeutic device insertion portion 28 is projecting provided on an outer peripheral surface of the circular cylindrical portion 114a2 of the insertion section connector 114. A suction-tube-equipped forceps valve (not shown) is mounted on the therapeutic device insertion portion 28. A therapeutic device, such as a forceps, is inserted from the therapeutic device insertion portion 28 through the suction-tube-equipped forceps valve. Further, the inserted therapeutic device can be projected forward from the distal-end opening portion 18 at the distal-end-side front surface of the insertion section 10 through the therapeutic device insertion conduit 26.

One end portion of a separate suction tube is coupled to the suction-tube-equipped forceps valve.
The therapeutic device insertion conduit 26 is coupled to the separate suction tube via the suction-tube-equipped forceps valve of the therapeutic device insertion portion 28. Thereby, the therapeutic device insertion conduit 26 is used also as a passage of sucked matter at a time of suction. In addition, the sucked matter can be sucked to the suction tube via the suction-tube-equipped forceps valve of the therapeutic device insertion portion 28 from the therapeutic device insertion conduit 26.

The power unit 11 includes, for example, a substantially circular cylindrical or substantially circular columnar unit body 11a. One end portion of the unit body 11a (a connection terminal portion for connection to the insertion section 10) is provided with an attachment portion 11b for attachment to the inner ring portion 107a of the endoscope holding section 104 of the endoscope arm 9. The attachment portion 11b is formed to have a shape corresponding to the inner ring portion 107a of the C-shaped ring 107. The attachment portion 11b of the power unit 11 is fixed in the state in which the attachment portion 11b is attached to the inner ring portion 107a of the C-shaped ring 107 of the endoscope arm 9. Thereby, the endoscope 2 is supported by the endoscope arm 9 so as to be movable in a predetermined range.

The power unit 11 includes a driving force generating section 11A having an electric motor (bend-driving means) 35, which is a driving source for driving the bending section 14 by motor driving; a light transmission section 11B which transmits illumination light by a light guide 125; and an electric signal transmission section 11C which transmits an electric signal of the CCD 22.

The driving force generating section 11A comprises a motor control unit 36 which executes an overall control of the power unit 11 including the electric motor 35; an encoder 37 which generates data indicative of the operation state of the electric motor 35, such as a rotation speed and a rotation amount; a deceleration gear 38 which decelerates the rotational driving force of the electric motor 35; an electromagnetic clutch 40 which is coupled to the deceleration gear 38 and transmits the rotational force of the electric motor 35 to a force coupling unit 39 on the insertion section 10 side; a potentiometer 41 which is rotational position detection means; a clutch operation detection switch 42 which detects the operation of the electromagnetic clutch 40; and an attachment/detachment state detection switch 43 which detects the engagement state between the insertion section 10 and the power unit 11.

Further, in the unit body 11a, an opening portion 11d of a reception chamber 11c, in which the part of the third block 114c of the insertion section connector 114 is detachably inserted, is formed in an end face on the attachment portion 11b side.

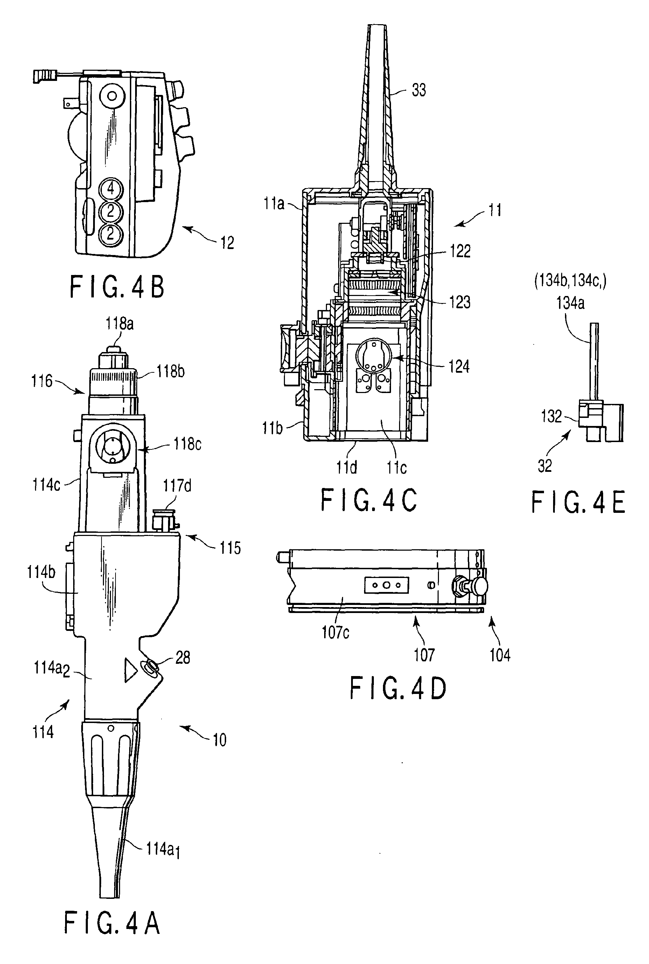

In the inside of the reception chamber 11c of the unit body 11a, as shown in FIG. 4C, there are provided a light guide connection portion 122 for optical connection, which forms the light transmission section 11B; electric contacts 123 for electrical connection, which constitute the electric signal transmission section 11C; and coupling portions 124 for driving force transmission. The light guide connection portion 122, electric contacts 123 and coupling portions 124 are disposed at such positions that the light guide connection portion 122, electric contacts 123 and coupling portions 124 are connected to the connection terminal portion 21a of the light guide 21 of the insertion section connector 114, the electric contacts 120 and the coupling portions 121a and 121b, respectively, when the part of the third block 114c of the insertion section connector 114 is detachably inserted in the reception chamber 11c of the unit body 11a and the insertion section connector 114 is inserted to the terminal end position of the reception chamber 11c. The light guide connection portion 122, electric contacts 123 and coupling portions 124 constitute a second external device connector for detachable connection to the second connector portion 116 of the insertion section connector 114.

One end portion of a universal cord 33 is coupled to a rear end portion of the unit body 11a. The light guide 125 and an electric line 126 for transmitting an electric signal of the CCD 22 are disposed in the universal cord 33. An optical connector portion 127, which is connected to the light source device 3, is coupled to a distal end portion of the universal cord 33. An electrical connector portion 129 is coupled to a side part of the optical connector portion 127 via an electric cable 128. The electrical connector portion 129 is connected to the video processor 4.

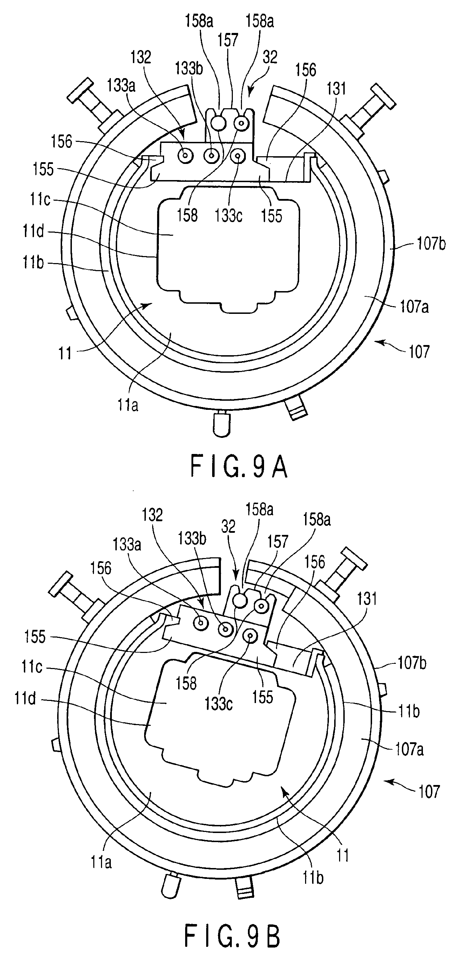

As shown in FIG. 5 and FIGS. 9A and 9B, a flat-plate-shaped notch portion 130 is formed on the unit body 11a by making a notch in a part of a circular peripheral surface of the unit body 11a. A conduit connector mounting portion 131 is provided on the notch portion 130 on the connection terminal side for connection to the insertion section 10. The conduit connector 32 is detachably mounted on the conduit connector mounting portion 131.

As shown in FIG. 9A, the conduit connector 32 has a block-shaped connector body 132 which is formed of an elastic material such as rubber. Three (first, second and third) connection hole portions 133a, 133b and 133c for connection to fluid conduits are formed in the connector body 132. One end portion of a forward water-feed tube 134a is coupled to the first connection hole portion 133a, one end portion of a water-feed tube 134b is coupled to the second connection hole portion 133b, and one end portion of an air-feed tube 134c is coupled to the third connection hole portion 133c.

The first, second and third connection hole portions 133a, 133b and 133c of the conduit connector 32 are formed of female mouthpieces in which the three male mouthpieces projecting from the first connector portion 115 of the insertion section connector 114, namely, the forward water-feed mouthpiece 117a, water-feed mouthpiece 117b and air-feed mouthpiece 117c, are to be inserted. The first, second and third connection hole portions 133a, 133b and 133c include engagement holes (not shown) for engagement, which have greater diameters than the outside diameters of the mouthpieces from 117a to 117c that are male mouthpieces, and projection portions for sealing, which are inwardly projectingly provided on the inner peripheral surface of the engagement holes. Each projection portion is so provided as to extend in the circumferential direction over the entire inner peripheral surface of the engagement hole. The inside diameters of the projection portions are less than the outside diameters of the mouthpieces from 117a to 117c that are male mouthpieces. Accordingly, when the mouthpieces from 117a to 117c that are male mouthpieces are inserted in the engagement holes, the parts of the projection portions are pressed and elastically deformed by the mouthpieces from 117a to 117c, and the gaps between the mouthpieces from 117a to 117c and the inner peripheral surfaces of the engagement holes are watertightly sealed.

Projection portions 155 are projectingly provided on both sides of the connector body 132 of the conduit connector 32 at the lower part of the connector body 132. As shown in FIG. 9A and FIG. 9B, the projection portions 155 are configured to be engaged with inverted-L-shaped engaging projection portions 156 which are upwardly projectingly provided on the bottom surface of the conduit connector mounting portion 131 of the power unit 11. Thereby, the conduit connector 32, which is mounted on the conduit connector mounting portion 131 of the power unit 11, is prevented from being pulled out to the upper side in FIG. 9A (i.e. in a direction perpendicular to the direction of removal of the insertion section 10).

Further, a tube-like holding member 157 is projectingly provided at an upper part of the connector body 132 of the conduit connector 32. Two tube-like component holding grooves 158 are formed in the tube-like holding member 157. A slot-shaped slit portion 158a is formed on an upper side of each tube-like component holding groove 158. A tube-like component, such as a tube or a wire, can detachably be inserted in the tube-like component holding groove 158 from the slit portion 158a.

Before the insertion section connector 114 of the insertion section 10 is coupled to the power unit 11, the conduit connector 32 is mounted on the conduit connector mounting portion 131 of the power unit 11 in advance. When the power unit 11 and the conduit connector 32 are connected, each of the other ends of the forward water-feed tube 134a, water-feed tube 134b and air-feed tube 134c of the conduit connector 32 is connected to the pump unit 5. At this time, the universal cord 33, the forward water-feed tube 134a, water-feed tube 134b and air-feed tube 134c of the conduit connector 32, and a suction tube 161 are inserted, together with a suction tube (not shown), through the bundle retainers 106 of the first support rod 105a and second support rod 105b so as to be axially movable and to be pivotable/rotatable about the axis.

A fluid control cassette 5a is detachably mounted on the pump unit 5. The fluid control cassette 5a includes a flow amount adjusting mechanism having valves associated with air feed, water feed and suction. The pump unit 5 drives the flow amount adjusting mechanism of the fluid control cassette 5a.

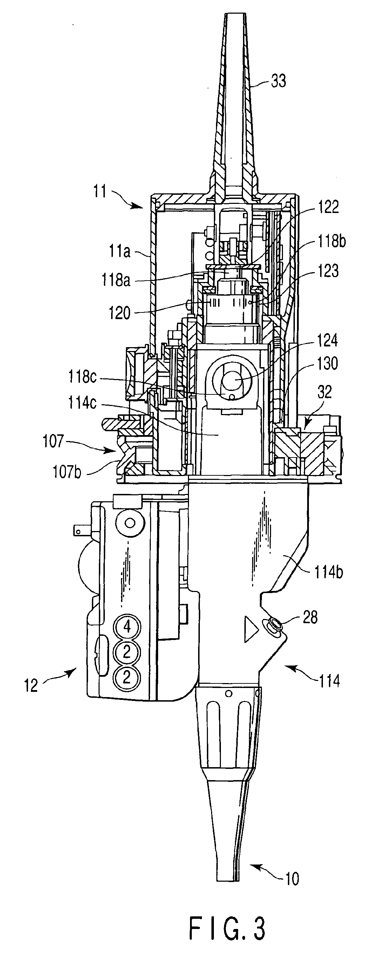

After the conduit connector 32 is mounted on the conduit connector mounting portion 131, the insertion connector 114 of the insertion section 10 is coupled to the power unit 11, as shown in FIG. 3. At this time, the optical connection section 118a, electrical connection section 118b and driving force transmission section 118c for driving force transmission of the second connector section 116 of the insertion section connector 114 are connected to the light guide connection portion 122, electric contacts 123 and coupling portions 124, which are the second external device connector of the power unit 11. Thereby, the light guide 21 for light transmission, the electric line 113 for electric signal transmission and the coupling portions 121a and 121b for driving force transmission, which are included in the insertion section 10, are connected, respectively, to the light guide 125, electric line 126 and coupling portions 124, which are included in the power unit 11.

At the same time, the first connector portion 115 of the insertion section connector 114 of the insertion section 10 is coupled to the conduit connector 32. At this time, the forward water-feed mouthpiece 117a, water-feed mouthpiece 117b and air-feed mouthpiece 117c of the first connector portion 115 are coupled to the first, second and third connection hole portions 133a, 133b and 133c of the conduit connector 32. Thereby, the forward water-feed conduit 24, air-feed conduit 25a and water-feed conduit 25b on the insertion section 10 side are respectively connected to the forward water-feed tube 134a, water-feed tube 134b and air-feed tube 134c on the conduit connector 32 side.

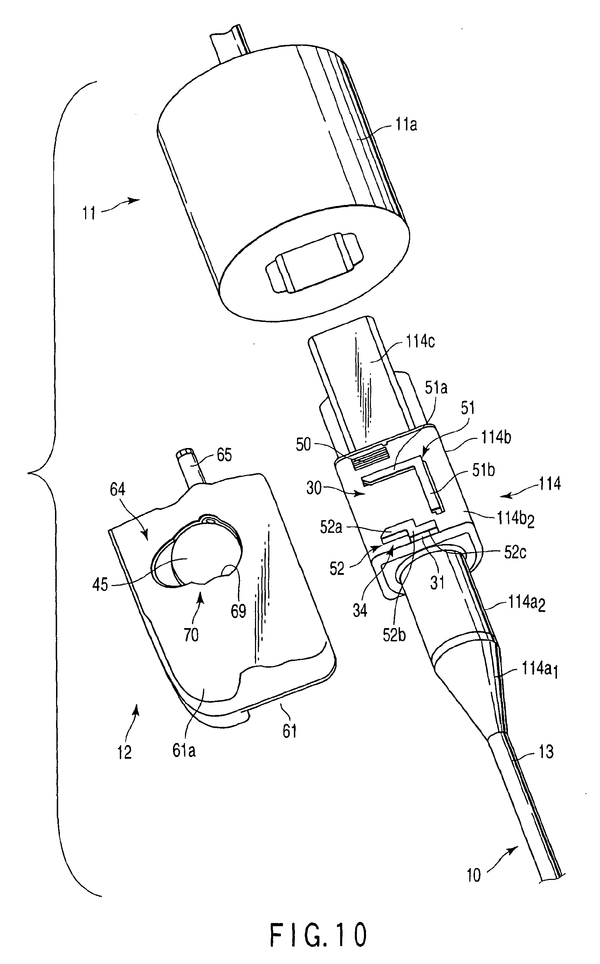

As shown in FIG. 10, an operation section attachment portion 30 is formed on one side surface 114b2 of the second block 114b of the insertion section connector 114. The operation section attachment portion 30 includes an engaging portion 31, guide means 34 and a fixing rib (fixing portion) 50. The engaging portion 31 detachably engages the operation section unit 12. The guide means 34 guides the operation section unit 12 to the position of the engaging portion 31. The fixing rib 50 fixes the operation section unit 12 at the position of the engaging portion 31.

The guide means 34 includes two (first and second) guide rail structure members 51 and 52 which are projectingly provided on the side surface 114b2 of the second block 114b. The first guide rail structure member 51 includes two (first and second) guide rails (guide portions) 51a and 51b which are bent substantially in an L shape. The first guide rail 51a extends in a direction different from the direction in which the proximal end portion of the insertion section 10 is detachably attached to the power unit 11, for example, in a direction perpendicular to the direction of attachment/detachment. The second guide rail 51b is coupled to a terminal end position of the first guide rail 51a, and extends in a direction different from the direction of the first guide rail 51a, for example, in a direction perpendicular to the direction of extension of the first guide rail 51a.

The second guide rail structure member 52 includes two (first and second) guide rails (guide portions) 52a and 52b which are bent substantially in an L shape, and one terminal end fixing portion 52c. The first guide rail 52a extends in parallel to the first guide rail 51a of the first guide rail structure member 51. The second guide rail 52b extends in parallel to the second guide rail 51b of the first guide rail structure member 51. One end portion of the second guide rail 52b is coupled to the terminal end position of the first guide rail 52a, and the terminal end fixing portion 52c is coupled to the other end portion of the second guide rail 52b. The engaging portion 31 for engagement with the operation section unit 12 is constituted by the terminal end fixing portion 52c.

When the operation section unit 12 is to be attached to the operation section attachment portion 30, the operation section unit 12 is first guided in a first guide direction by the first guide rail 51a of the first guide rail structure member 51 and the first guide rail 52a of the second guide rail structure member 52. Then, the operation section unit 12 is guided in a second guide direction by the second guide rail 51b of the first guide rail structure member 51 and the second guide rail 52b of the second guide rail structure member 52. Thereby, the operation section unit 12 is guided in two different directions and is guided to the position of the terminal end fixing portion 52c. In the meantime, the second guide direction, in which the operation section unit 12 is guided by the second guide rail 51b of the first guide rail structure member 51 and the second guide rail 52b of the second guide rail structure member 52, is set to be such a direction as to have resistance against the direction of twisting of the insertion section 10 about its center axis.

The operation section unit 12 is used in at least one of a state (see FIG. 3) in which the operation section unit 12 is attached to the operation section attachment portion 30 of the insertion section 10 and a state (see FIG. 1 and FIG. 2) in which the operation section unit 12 is removed from the operation section attachment portion 30 of the insertion section 10. From FIG. 10 to FIG. 13 show the state in which the operation section unit 12 is removed from the operation section attachment portion 30 of the insertion section 10.

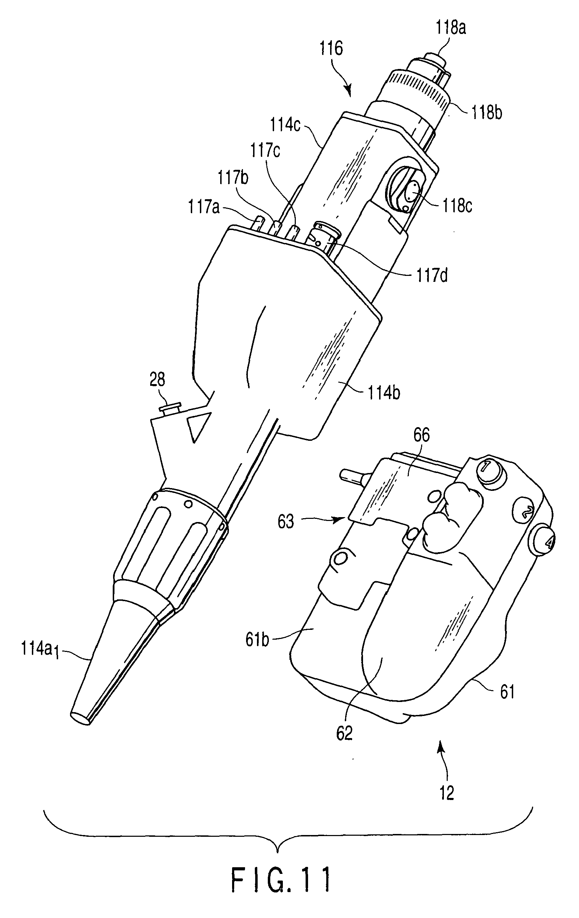

The operation section unit 12 includes an operation section unit body 61. As shown in FIG. 10, a trackball 45, which is a bending operation input device, is disposed on a front surface 61a of the operation section unit body 61. As shown in FIG. 11, a grip portion (grasping portion) 62, which can be grasped by the operator, and an attachment/detachment portion 63 for attachment/detachment to/from the operation section attachment portion 30 of the insertion section 10 are provided on a back surface 61b of the operation section unit body 61.

Further, the operation section unit 12 is provided with an operation input section 64 for operating the endoscope 2, and an attachment/detachment operation lever (rotational lever) 65. As shown in FIG. 2, the operation input section 64 includes the trackball 45, various operation members such as an air-feed/water-feed switch 46 and a suction switch 47, and scope switches 48. The air-feed/water-feed switch 46 instructs an air-feed/water-feed operation. The suction switch 47 instructs a suction operation. The scope switches 48 are various video switches which execute remote-control of various functions of the video processor 4, such as video imaging.

The operation section unit body 61 is provided with, for instance, an AD converter 49 which is electrically connected to the operation input section 64. One end of an electric cable 44 is connected to the operation section unit 12. The other end of the electric cable 44 is connected to the pump unit 5. The AD converter 49 receives electric signals which are generated from the various scope switches 48 that execute operations of video imaging, etc., and executes an AD conversion process for converting the electric signals to predetermined operation instruction signals. Switch functions, such as a freeze (still image) instruction and (release), can be assigned to the individual plural scope switches 48 of the operation section unit 12. By the switch operation of the scope switch 48, the signal process corresponding to the assigned function, for example, an operation of outputting a still image to the display screen of the monitor 8, is performed.

When the operation members of the operation section unit 12 are operated, various instruction signals are generated by the operation of the respective operation members. The AD converter 49 properly outputs control signals for executing controls corresponding to these instruction signals to the respective devices. Thereby, the driving control of the power unit 11 is performed, and an overall control of the light source device 3, video processor 4 and pump unit 5 etc. is executed.

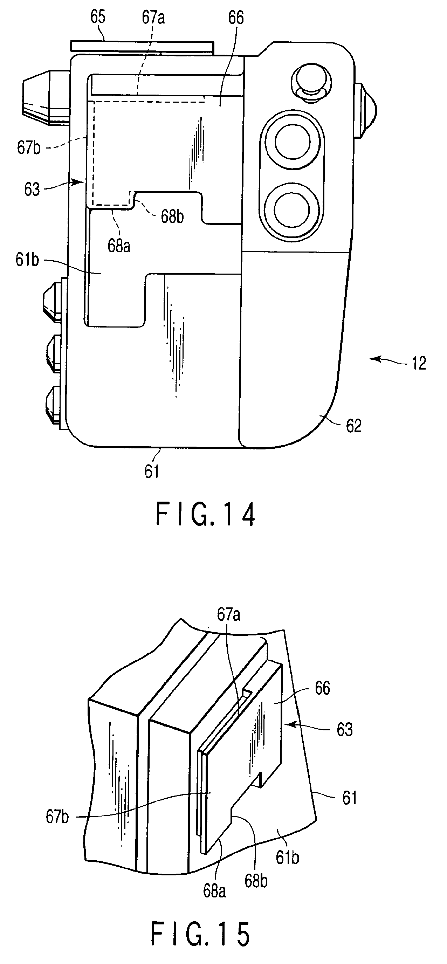

As shown in FIG. 14 and FIG. 15, the attachment/detachment portion 63 of the operation section unit 12 includes a flat-plate-shaped block 66. The flat-plate-shaped block 66 is projectingly provided on the back surface 61b of the operation section unit body 61. First and second slide ribs 67a and 67b having groove shapes are formed at peripheral edge parts of the block 66. The first and second slide ribs 67a and 67b are disposed at positions corresponding to the first and second guide rails 51a and 51b of the first guide rail structure member 51 of the operation section attachment portion 30.

Similarly, first and second slide ribs 68a and 68b having groove shapes are formed at peripheral edge parts of the block 66. The first and second slide ribs 68a and 68b are disposed at positions corresponding to the first and second guide rails 52a and 52b of the second guide rail structure member 52.

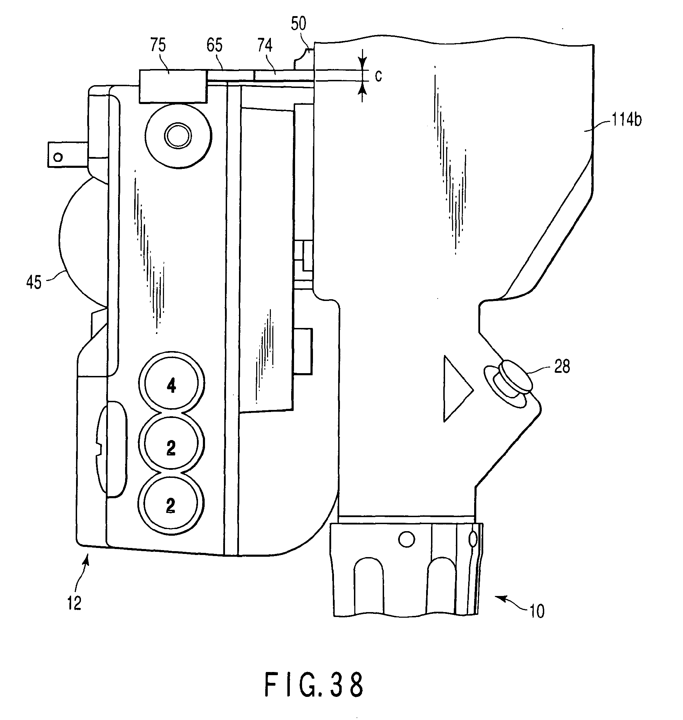

At a time of the work for mounting the attachment/detachment portion 63 of the operation section unit 12 to the operation section attachment portion 30 of the insertion section 10, the first slide ribs 67a and 68a of the attachment/detachment portion 63 are respectively moved along the first guide rail 51a of the first guide rail structure member 51 of the operation section attachment portion 30 and the first guide rail 52a of the second guide rail structure member 52. Subsequently, the second slide ribs 67b and 68b of the attachment/detachment portion 63 are respectively moved along the second guide rail 51b of the first guide rail structure member 51 of the operation section attachment portion 30 and the second guide rail 52b of the second guide rail structure member 52. Then, the first slide rib 68a of the attachment/detachment portion 63 is detachably engaged with the engaging portion 31 of the operation section attachment portion 30. At this time, as shown in FIG. 38, a clearance C is formed between the fixing rib 50 and the attachment/detachment portion 63 of the operation section unit 12. Thereby, the second slide ribs 67b and 68b of the attachment/detachment portion 63 of the operation section unit 12 are respectively movable along the second guide rail 51b of the first guide rail structure member 51 of the operation section attachment portion 30 and the second guide rail 52b of the second guide rail structure member 52.

A trackball mounting portion 70 having a recess portion 69 for attachment of the trackball 45 is formed on the front surface 61a of the operation section unit body 61. The trackball 45 is rotatably and detachably mounted in the trackball mounting portion 70.

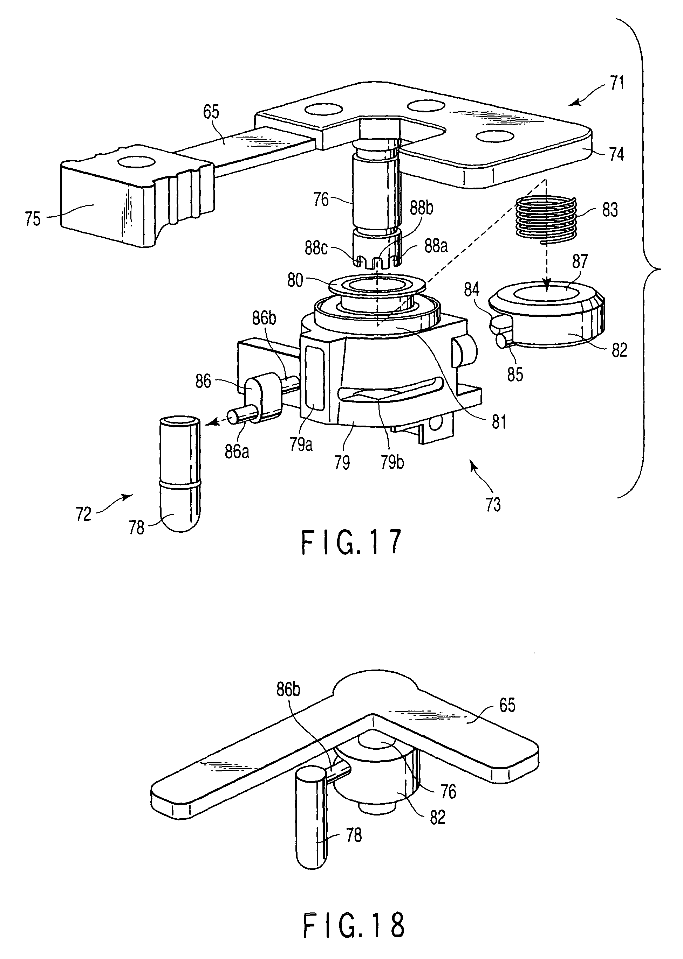

As shown in FIG. 17, the operation section unit body 61 is provided with operation section engaging means 71, removal restriction means 72 and interlock drive means 73. When the operation section unit 12 is attached to the operation section attachment portion 30, the operation section engaging means 71 detachably engages the operation section unit 12 with the operation section attachment portion 30. The removal restriction means 72 restricts removal of the trackball 45, which is mounted in the trackball mounting portion 70, from the trackball mounting portion 70. The interlock drive means 73 is coupled to the operation section engaging means 71 and removal restriction means 72 and interlockingly drives the operation section engaging means 71 and removal restriction means 72.

The operation section engaging means 71 includes a plate-shaped member 74 which is disposed at one end portion of the attachment/detachment operation lever 65 of the operation section unit 12. The attachment/detachment operation lever 65 is formed substantially in an L shape. The plate-shaped member 74 is provided at one end portion of the L-shaped attachment/detachment operation lever 65, and an operation knob 75 is provided at the other end portion of the L-shaped attachment/detachment operation lever 65.

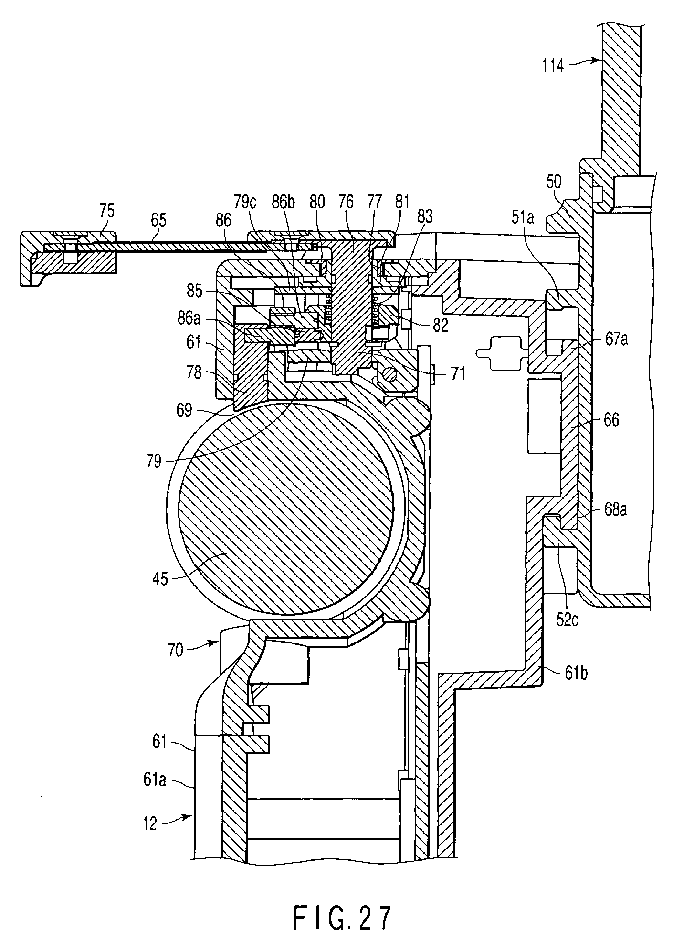

An upper end portion of a rotational shaft 76 is fixed to an L-shaped bent part of the attachment/detachment operation lever 65. As shown in FIG. 27, the rotational shaft 76 is rotatably inserted in a shaft hole 77 which is formed in an end face of the operation section unit body 61. The attachment/detachment operation lever 65 is supported to be rotatable about the rotational shaft 76.

When the operation section unit 12 is attached to the operation section attachment portion 30, the attachment/detachment lever 65 is rotated about the rotational shaft 76 in the state in which the first slide rib 68a of the attachment/detachment portion 63 is detachably engaged with the engaging portion 31 of the operation section attachment portion 30. Thus, as shown in FIG. 38, the plate-shaped member 74 is engaged with the fixing rib 50 in the state in which the plate-shaped member 74 of the attachment/detachment operation lever 65 is inserted in the clearance C that is formed between the fixing rib 50 and the attachment/detachment portion 63 of the operation section unit 12.
Thereby, the operation section unit 12 restricts the movement of the second slide ribs 67b and 68b of the attachment/detachment portion 63 along the second guide rail 51b of the first guide rail structure member 51 of the operation section attachment portion 30 and the second guide rail 52b of the second guide rail structure member 52 in the range of the clearance C.

The removal restriction means 72 includes a stopper pin 78 which is advancible/retreatable in the recess portion 69 of the trackball mounting portion 70 in accordance with the operation of the interlock drive means 73. The stopper pin 78 is engaged with the trackball 45, thereby preventing the trackball 45 from being removed and falling from the trackball mounting portion 70.

FIG. 17 shows the structure of the interlock drive means 73. The interlock drive means 73 includes a bracket 79 in which the rotational shaft 76 of the attachment/detachment operation lever 65 is fitted in. An upper bearing member 80 and a lower bearing member 81 are provided on the upper surface of the bracket 79. A vertical groove 79a and a horizontal groove 79b are provided on an outer peripheral surface of the bracket 79. The vertical groove 79a is formed of a long hole extending in the same direction as the axial direction of the rotational shaft 76. The horizontal groove 79b is formed of a long hole extending in a direction perpendicular to the vertical groove 79a.

As shown in FIG. 27, a wheel 82 and a coil spring 83 are provided in the bracket 79. The wheel 82 is integrally fastened to the rotational shaft 76 of the attachment/detachment operation lever 65 by a screw. A wheel groove 84 is circumferentially extendingly provided on the outer peripheral surface of the wheel 82, and a projection portion 85 is projectingly provided at one end portion of the wheel groove 84. The projection portion 85 is inserted in the horizontal groove 79b of the bracket 79.

A coupling shaft 86 is inserted in the vertical groove 79a of the bracket 79 so as to be movable along the vertical groove 79a in the vertical direction in FIG. 17. An outer pin 86a is projectingly provided on an outer end portion of the coupling shaft 86. An inner pin 86b is projectingly provided on an inner end portion of the coupling shaft 86. The outer pin 86a is fixed to the stopper pin 78. The inner pin 86b is inserted in the wheel groove 84 of the wheel 82.

Further, a spring-receiving recess portion 87 is formed in an upper surface of the wheel 82. A lower end portion of the coil spring 83 is received in the recess portion 87. An upper end portion of the coil spring 83 is abutted upon an upper surface plate 79c of the bracket 79. Thereby, the stopper pin 78 always receives a load in such a direction as to engage the trackball 45 from the coil spring 83 via the wheel 82 that is interlocked with the attachment/detachment operation lever 65, thus preventing removal of the trackball 45.

In the present embodiment, a distal end portion of the stopper pin 78 has such a shape as to trace a spherical surface R of the trackball 45. However, the distal end portion of the stopper pin 78 may have any geometrical shape if it can hold the trackball 45.

The stopper pin 78 is supported so as to be movable along the vertical groove 79a of the bracket 79 via the coupling shaft 86. The inner pin 86b of the coupling shaft 86 is supported so as to be movable along the wheel groove 84 of the wheel 82. Thereby, the movement of the stopper pin 78 in the vertical direction is restricted.

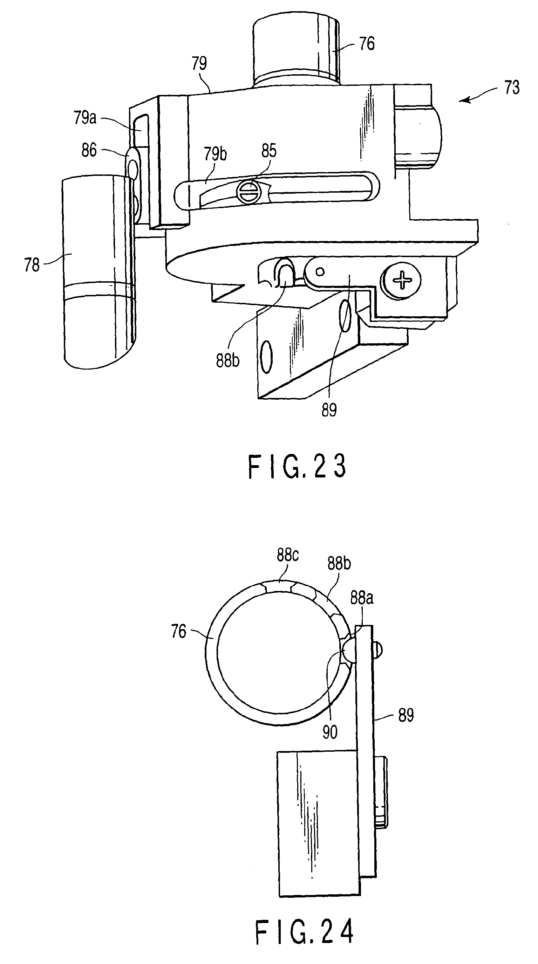

As shown in FIG. 24, a plurality (three in the present embodiment) of engaging grooves 88a, 88b and 88c are formed at a lower end portion of the rotational shaft 76. The three engaging grooves 88a, 88b and 88c are disposed in the circumferential direction of the rotational shaft 76 at intervals of 45°.

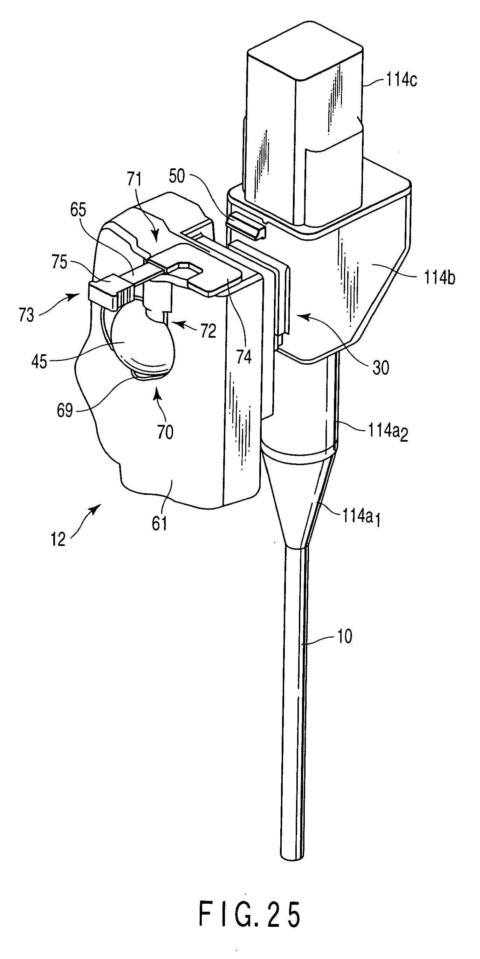

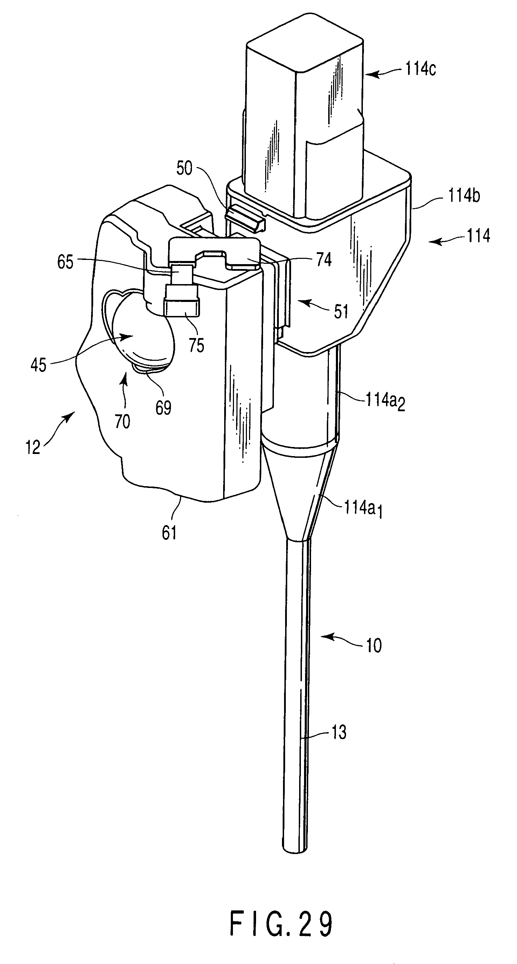

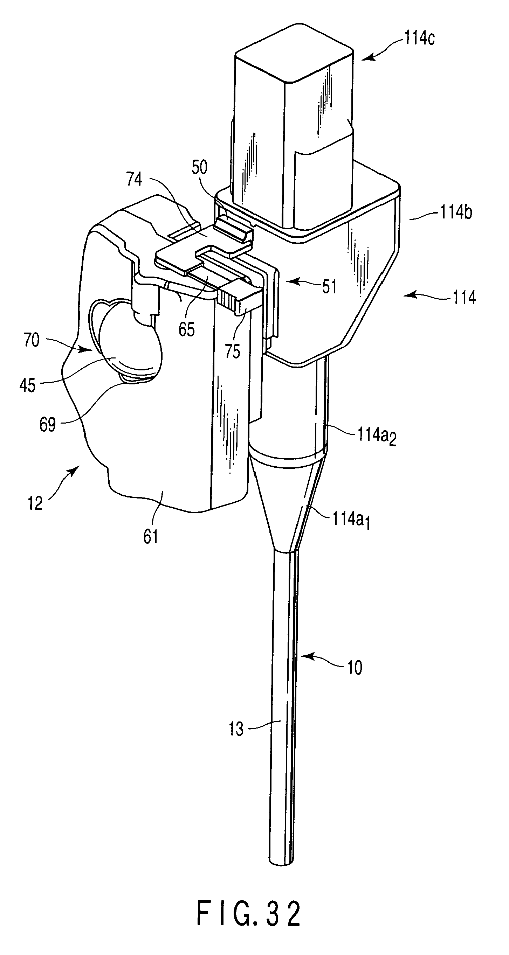

Further, a plate spring 89 is attached to a lower end portion of the bracket 79 at a position corresponding to a lower end portion of the rotational shaft 76. An engaging pin 90 is fixed to a distal end portion of the plate spring 89. The engaging pin 90 is configured to be detachably selectively engaged with the three engaging grooves 88a, 88c and 88c of the rotational shaft 76. Thereby, when the rotational shaft 76 is rotated, a sensation of clicking is created by the engagement/disengagement movement between the engaging pin 90 at the distal end of the plate spring 89 and the engaging grooves 88a, 88c and 88c.
Moreover, the rotation of the rotational shaft 76 is restricted by a definite amount at a rotational position of 0° (unlocked state shown in from FIG. 25 to FIG. 28), a rotational position of 45° (intermediated locked state shown in from FIG. 29 to FIG. 31) and a rotational position of 90° (lock position shown in from FIG. 32 to FIG. 36). Thereby, the attachment/detachment operation lever 65 can be held at the rotational position of 90° (lock position). In the meantime, the method of holding the attachment/detachment operation lever 65 is not limited to this.

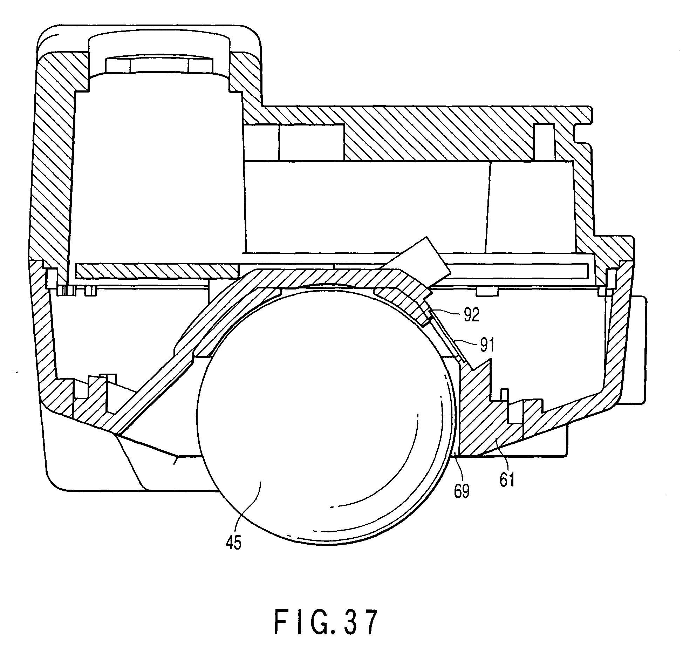

In the present embodiment, as shown in FIG. 37, a part of the trackball mounting portion 70 of the operation section unit body 61 is provided with a glass window 91 for permeation of a laser beam of a laser device for scanning the trackball 45. The glass window 91 is disposed in a direction perpendicular to a line extending through the center of the trackball 45. The glass window 91 is watertightly attached to the peripheral wall portion of the trackball mounting portion 70 via an adhesive portion 92.

Next, the operation of the above-described structure is described. When the system of the motor-driven bending endoscope 2 of the present embodiment is used, a work (attachment/detachment) for attaching/detaching the conduit connector 32 and the insertion section 10 to/from the power unit 11 is performed. At this time, the conduit connector 32 is first connected to the conduit connector mounting portion 131 of the power unit 11. Then, a work for coupling the insertion section 10 to the power unit 11 is performed.

At the time of this work, the insertion section connector 114 of the insertion section 10 is inserted in the reception chamber 11c from the opening portion 11d at the end face on the attachment portion 11b side of the power unit 11. At this time, the optical connection section 118a, electrical connection section 118b and driving force transmission section 118c for driving force transmission of the second connector portion 116 of the insertion section connector 114 are connected to the light guide connection portion 122, electric contacts 123 and coupling portions 124, which are the second external device connector of the power unit 11. Thereby, the light guide 21 for light transmission, the electric line 113 for electric signal transmission and the coupling portions 121a and 121b for driving force transmission, which are included in the insertion section 10, are connected, respectively, to the light guide 125, electric line 126 and coupling portions 124, which are included in the power unit 11.

If the power unit 11 and the insertion section 10 are coupled, the conduit connector 32 is coupled, at almost the same time, to the forward water-feed mouthpiece 117a, water-feed mouthpiece 117b and air-feed mouthpiece 117c of the first connector portion 115 of the insertion section 10. At this time, the forward water-feed mouthpiece 117a, water-feed mouthpiece 117b and air-feed mouthpiece 117c of the first connector portion 115 are coupled to the first, second and third connection hole portions 133a, 133b and 133c of the conduit connector 32. Thereby, the forward water-feed conduit 24, air-feed conduit 25a and water-feed conduit 25b on the insertion section 10 side are connected to the forward water-feed tube 134a, water-feed tube 134b and air-feed tube 134c on the conduit connector 32 side.

In the present embodiment, the operation section unit 12 is usable in an independent state in which the operation section unit 12 is separated from the operation section attachment portion 30 of the insertion section 10, as shown in FIG. 1 and FIG. 2, and is also usable in a state in which the operation section unit 12 is attached to the operation section attachment portion 30 of the insertion section 10, as shown in FIG. 3. The user can use the operation section unit 12, as the need arises, in one of the independent state in which the operation section unit 12 is separated from the operation section attachment portion 30 of the insertion section 10 and the state in which the operation section unit 12 is attached to the operation section attachment portion 30 of the insertion section 10.

In the case where the operation section unit 12 is attached to the operation section attachment portion 30 of the insertion section 10, the following operation is performed. To begin with, the rotational shaft 76 of the attachment/detachment operation lever 65 of the operation section unit 12 is set at the rotational position of 0° (unlocked state shown in from FIG. 25 to FIG. 28). When the rotational shaft 76 of the attachment/detachment operation lever 65 of the operation section unit 12 is at the rotational position of 0° (unlocked state shown in from FIG. 25 to FIG. 28), the stopper pin 78 is held in the state in which the stopper pin 78 is movable in interlock with the attachment/detachment operation lever 65 in the vertical direction (i.e. in the direction in which the coupling shaft 86 is movable along the vertical groove 79a). At this time, the stopper pin 78 always receives a load from the coil spring 83 via the wheel 82 in such a direction as to engage the trackball 45 in the recess portion 69 of the trackball mounting portion 70. Thus, unless the stopper pin 78 is moved, the removal of the trackball 45 from the recess portion 69 of the trackball mounting portion 70 can be suppressed.

If the trackball 45 is pulled out against the spring force of the coil spring 83 or the attachment/detachment operation lever 65 is pulled up in the state in which the rotational shaft 76 of the attachment/detachment operation lever 65 is at the rotational position of 0°, the stopper pin 78 is moved in such a direction as to be disengaged from the engaging position in the recess portion 69 of the trackball mounting portion 70, and the trackball 45 can be removed from the recess portion 69 of the trackball mounting portion 70.

Subsequently, the attachment/detachment operation lever 65 is rotated in a direction of locking, and the rotational shaft 76 of the attachment/detachment operation lever 65 is moved to the rotational position of 45° (intermediated locked state shown in from FIG. 29 to FIG. 31). At this time, the projection portion 85 of the wheel 82, which moves in interlock with the attachment/detachment operation lever 65, is inserted in the horizontal groove 79b of the bracket 79, thereby restricting the movement of the stopper pin 78 in the vertical direction (i.e. in the direction in which the coupling shaft 86 moves along the vertical groove 79a). Thereby, the trackball 45 can be prevented from being removed from the recess portion 69 of the trackball mounting portion 70.

Subsequently, the work for attaching the operation section unit 12 to the operation section attachment portion 30 of the insertion section 10 is performed.
At the time of this work, the first slide ribs 67a and 68a of the attachment/detachment portion 63 of the operation section unit 12 are respectively moved along the first guide rail 51a of the first guide rail structure member 51 of the operation section attachment portion 30 and the first guide rail 52a of the second guide rail structure member 52 (the operation section unit 12 is guided in the first guide direction). When the attachment/detachment portion 63 of the operation section unit 12 is moved, the second slide rib 67b of the attachment/detachment portion 63 abuts on the second guide rail 51b of the first guide rail structure member 51 of the operation section attachment portion 30 at the terminal end position of the first guide rail 51a of the operation section attachment portion 30.

At this position, the direction of movement of the attachment/detachment portion 63 of the operation section unit 12 is changed about 90°. Then, the second slide ribs 67b and 68b of the attachment/detachment portion 63 are respectively moved along the second guide rail 51b of the first guide rail structure member 51 of the operation section attachment portion 30 and the second guide rail 52b of the second guide rail structure member 52 (the operation section unit 12 is guided in the second guide direction). Thereby, the operation section unit 12 is guided in the two different directions.

When the direction of movement of the attachment/detachment portion 63 of the operation section unit 12 is changed about 90°, the first slide rib 68a of the attachment/detachment portion 63 is detachably engaged with the engaging portion 31 of the operation section attachment portion 30 at the terminal end position of the second guide rail 52b of the second guide rail structure member 52 of the operation section attachment portion 30. At this time, as shown in FIG. 38, the clearance C is formed between the fixing rib 50 and the attachment/detachment portion 63 of the operation section unit 12.

In this state, the attachment/detachment operation lever 65 is further rotated in the direction of locking. The rotational shaft 76 of the attachment/detachment operation lever 65 is moved to the rotational position of 90° (lock position shown in from FIG. 32 to FIG. 36). If the attachment/detachment operation lever 65 is moved to the rotational position of 90°, the plate-shaped member 74 of the attachment/detachment operation lever 65 is inserted and hooked under the fixing rib 50 of the operation section attachment portion 30. This restricts the movement of the second slide ribs 67b and 68b of the attachment/detachment portion 63 of the operation section unit 12 in the second guide direction along the second guide rail 51b of the first guide rail structure member 51 of the operation section attachment portion 30 and the second guide rail 52b of the second guide rail structure member 52. As a result, the operation section unit 12 is fixed at the position of the engaging portion 31, and the work for attaching the operation section unit 12 to the operation section attachment portion 30 of the insertion section 10 is completed.

In the case where the operation section unit 12, which is attached to the operation section attachment portion 30 of the insertion section 10, is to be detached from the operation section attachment portion 30, the following operation is performed. To begin with, the attachment/detachment operation lever 65 of the operation section unit 12 is rotated in the direction of unlocking. At this time, if the rotational shaft 76 rotates from the rotational position of 90° to the rotational position of 45°, the plate-shaped member 74 of the attachment/detachment operation lever 65 is pulled out from under the fixing rib 50 of the operation section attachment portion 30. Thereby, the engagement between the plate-shaped member 74 of the attachment/detachment operation lever 65 and the fixing rib 50 of the operation section attachment portion 30 is released. In this state, as shown in FIG. 38, the second slide ribs 67b and 68b of the attachment/detachment portion 63 of the operation section unit 12 are respectively movable in the second guide direction along the second guide rail 51b of the first guide rail structure member 51 of the operation section attachment portion 30 and the second guide rail 52b of the second guide rail structure member 52 in the range of the clearance C between the fixing rib 50 and the attachment/detachment portion 63 of the operation section unit 12.

In this state, by moving the attachment/detachment portion 63 of the operation section unit 12 toward the fixing rib 50, the operation section unit 12 is moved away from the position of engagement with the engaging portion 31. Then, the second slide ribs 67b and 68b of the attachment/detachment portion 63 of the operation section unit 12 are moved in the second guide direction along the second guide rail 51b of the first guide rail structure member 51 of the operation section attachment portion 30 and the second guide rail 52b of the second guide rail structure member 52. At the time when the first slide rib 67a of the attachment/detachment portion 63 abuts on the first guide rail 51a of the first guide rail structure member 51 of the operation section attachment portion 30, the direction of movement of the attachment/detachment portion 63 of the operation section unit 12 is changed about 90°.

Subsequently, the first slide ribs 67a and 68a of the attachment/detachment portion 63 of the operation section unit 12 are respectively moved in the first direction along the first guide rail 51a of the first guide rail structure member 51 of the operation section attachment portion 30 and the first guide rail 52a of the second guide rail structure member 52. The operation section unit 12 is removed from the operation section attachment portion 30 by moving outward the attachment/detachment portion 63 of the operation section unit 12 from the beginning end portions of the first guide rail 51a of the first guide rail structure member 51 of the operation section attachment portion 30 and the first guide rail 52a of the second guide rail structure member 52. Since the attachment/detachment operation lever 65 of the operation section unit 12 is held at the rotational position of 45°, the stopper pin 78 is held in the state in which the movement of the stopper 78 is restricted. Thus, the trackball 45 can be prevented from being removed and falling from the recess portion 69 of the trackball mounting portion 70.

After the operation section unit 12 is removed from the operation section attachment portion 30, the attachment/detachment operation lever 65 is rotated to the rotational position of 90°. Thereby, the operation section unit 12 can be used in the independent state in which the operation section unit 12 is separated from the operation section attachment portion 30 of the insertion section 10. Furthermore, after the operation section unit 12 is removed from the operation section attachment portion 30, the trackball 45 can be removed from the recess portion 69 of the trackball mounting portion 70 by moving the stopper pin 78 in a direction of disengagement from the trackball 45 in the state in which the attachment/detachment operation lever 65 is rotated to the rotational position of 0°.

The following advantageous effects can be obtained with the above-described structure. In the present embodiment, the operation section unit 12 is detachably attached to the operation section attachment portion 30 at the proximal end portion of the insertion section 10 of the motor-driven bending endoscope 2. Accordingly, the operation section unit 12 is usable in the independent state in which the operation section unit 12 is separated from the operation section attachment portion 30 of the insertion section 10, as shown in FIG. 1 and FIG. 2, and is also usable in the state in which the operation section unit 12 is attached to the operation section attachment portion 30 of the insertion section 10, as shown in FIG. 3.

At the time of the work for attaching/detaching the operation section unit 12 to/from the operation section attachment portion 30, the operation section engaging means 71 and the removal restriction means 72 can interlockingly be driven by operating the attachment/detachment operation lever 65 of the interlock drive means 73. When the operation section unit 12 is attached to the operation section attachment portion 30, with the rotating operation of the attachment/detachment operation lever 65, the attachment/detachment portion 63 of the operation section unit 12 is detachably engaged with the operation section attachment portion 30 by the operation section engaging means 71, and the removal restriction means 72 can restrict removal of the trackball 45, which is mounted in the trackball mounting portion 70, from the trackball mounting portion 70. Therefore, the operation of detachably engaging the attachment/detachment portion 63 of the operation section unit 12 with the operation section attachment portion 30, and the operation of the removal restriction means 72 for restricting removal of the trackball 45, which is mounted in the trackball mounting portion 70, from the trackball mounting portion 70 can be performed at a time by the rotating operation of the single attachment/detachment operation lever 65 and the operation can be facilitated.

In addition, the operation of switching to the state in which the attachment/detachment portion 63 of the operation section unit 12 can be detached from the operation section attachment portion 30, and the operation of switching to the state in which the trackball 45 can be removed from the trackball mounting portion 70 of the operation section unit 12 can be performed at a time by the rotating operation of the attachment/detachment operation lever 65. Therefore, these operations can advantageously be facilitated.

In accordance with the angle of operation of the attachment/detachment operation lever 65, the timing of the operation of switching to the state in which the attachment/detachment portion 63 of the operation section unit 12 can be detached from the operation section attachment portion 30 is different from the timing of the operation of switching to the state in which the trackball 45 can be removed from the trackball mounting portion 70 of the operation section unit 12. In other words, if the attachment/detachment operation lever 65 is rotated 45° from the rotational position of 90° (lock position) to the rotational position of 45°, switching can be effected to the state in which the attachment/detachment portion 63 of the operation section unit 12 can be detached from the operation section attachment portion 30. If the attachment/detachment operation lever 65 is further rotated from the rotational position of 45° to the rotational position of 0° (i.e. in the state in which the attachment/detachment operation lever 65 is rotated 90° from the rotational position of 90°), switching can be effected to the state in which the trackball 45 can be removed from the trackball mounting portion 70 of the operation section unit 12. Thus, in the case where the attachment/detachment portion 63 of the operation section unit 12 is detached from the operation section attachment portion 30, the trackball 45 cannot be removed from the trackball mounting portion 70 of the operation section unit 12. Therefore, when the attachment/detachment portion 63 of the operation section unit 12 is detached from the operation section attachment portion 30, removal of the trackball 45 of the operation section unit 12 can surely be prevented.

The three engaging grooves 88a, 88b and 88c are disposed at intervals of 45° at the lower end portion of the rotational shaft 76 of the attachment/detachment operation lever 65, and the plate spring 89 is attached to the lower end portion of the bracket 79. The engaging pin 90 provided at the distal end portion of the plate spring 89 is configured to be detachably selectively engaged with the three engaging grooves 88a, 88c and 88c of the rotational shaft 76. Thereby, when the rotational shaft 76 is rotated, a sensation of clicking is created by the engagement/disengagement movement between the engaging pin 90 at the distal end of the plate spring 89 and the engaging grooves 88a, 88c and 88c.

Moreover, the rotation of the rotational shaft 76 is restricted by a definite amount at the rotational position of 0° (unlocked state shown in from FIG. 25 to FIG. 28), the rotational position of 45° (intermediated lock position shown in from FIG. 29 to FIG. 31) and the rotational position of 90° (lock position shown in from FIG. 32 to FIG. 36). Thereby, the attachment/detachment operation lever 65 can be held at the rotational position of 90° (lock position).

In the present embodiment, when the operation section unit 12 is to be attached to the operation section attachment portion 30, the operation section unit 12 is first guided in the first guide direction by the first guide rail 51a of the first guide rail structure member 51 and the first guide rail 52a of the second guide rail structure member 52. Then, the operation section unit 12 is guided in the second guide direction by the second guide rail 51b of the first guide rail structure member 51 and the second guide rail 52b of the second guide rail structure member 52. Thereby, the operation section unit 12 is guided to the position of the terminal end fixing portion 52c. At this time, since the first guide direction is set to be perpendicular to the direction of attachment/detachment of the proximal end portion of the insertion section 10 to/from the power unit 11, it is possible to reduce the risk of removal of the operation section unit 12 in association with the operation of attachment/detachment of the proximal end portion of the insertion section 10 to/from the power unit 11. Moreover, since the operation section unit 12 is guided in the two different directions, the risk of removal of the operation section unit 12 can further be reduced.

The second guide direction, in which the operation section unit 12 is guided by the second guide rail 51b of the first guide rail structure member 51 and the second guide rail 52b of the second guide rail structure member 52, is set to be such a direction as to have resistance against the direction of twisting of the insertion section 10 about its center axis. Thereby, since the structure that is robust to the twisting operation of the insertion section 10 can be obtained, there is an advantage that the insertion section 10 can properly be twisted about its axis while the motor-driven bending endoscope 2 is being used.

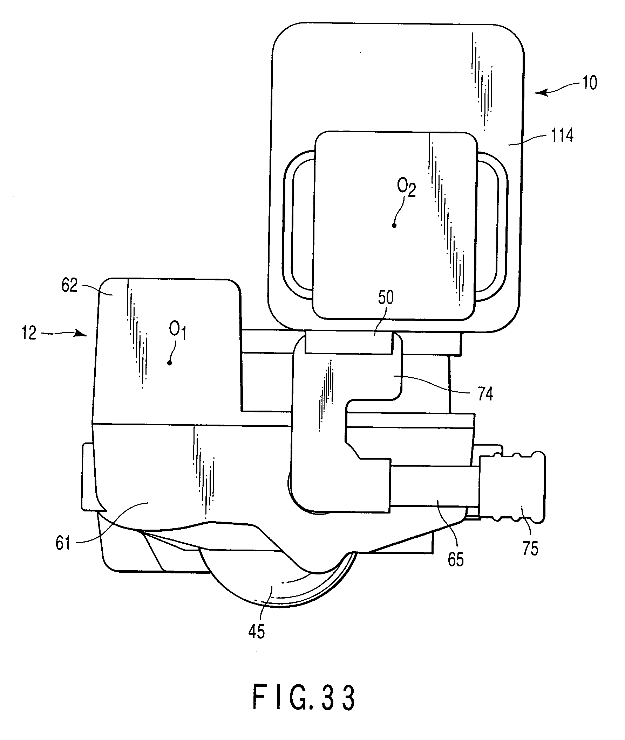

In the present embodiment, as shown in FIG. 33, a first center axis O1 that is the center of grasping of the grip portion 62 at the time of grasping the operation section unit 12 is positioned to be eccentric to a second center axis 02 that is the center axis of the insertion section 10. Thereby, when the operation section unit 12 is attached to the operation section attachment portion 30, the operation section unit 12 is offset from the center axis 02 of the insertion section 10, and the twisting operation is facilitated. Therefore, when the operation section unit 12 is used by selecting the state in which the operation section unit 12 is attached to the operation section attachment portion 30 of the insertion section 10, the fatigue of the right hand at the time of performing the twisting operation of the insertion section 10 can be reduced.

Besides, by using the operation section unit 12 in the state in which the operation section unit 12 is attached to the operation section attachment portion 30 of the insertion section 10, it becomes possible to prevent the occurrence of such a situation that the up-and-down direction of the insertion section 10 is not understandable by feeling. Thus, the recognizability of the up-and-down direction at the time of insertion of the insertion section 10 can be enhanced, and the operational work efficiency can be improved.

In the present embodiment, the removal restriction means 72 for restricting removal of the trackball 45 includes the stopper pin 78 which is advancible/retreatable in the recess portion 69 of the trackball mounting portion 70 in accordance with the operation of the interlock drive means 73. The stopper pin 78 is engaged with the trackball 45, thereby preventing the trackball 45 from being removed and falling from the trackball mounting portion 70. Accordingly, when the operation section unit 12 is operated, for example, when the operation section unit 12 is twisted or when the operation section unit 12 is carried in the hospital, the trackball 45 can be prevented from being removed and falling from the operation section unit 12. In addition, at a time of a reprocess, the trackball 45 can easily be removed from the operation section unit 12. As a result, both the prevention of removal of the trackball 45 and the improvement of the reprocess performance of the operation section unit 12 can be achieved at the same time.

Furthermore, the operation section unit 12 is configured to be detachably attached to the operation section attachment portion 30 at the proximal end portion of the insertion section 10 of the motor-driven endoscope 2. By virtue of this configuration, the operation section unit 12 may be used as a separate body when the merit of use of the operation section 12 as a separate body is to be obtained, and the operation section unit 12 may be used as an integrally attached body when the merit of use of the operation section 12 as an integrally attached body is to be obtained. Consequently, it is possible to obtain the merit of use of the operation section 12 both as a separate body and as an integrally attached body.

The present invention is not limited to the above-described embodiment and, needless to say, the invention can be various modified and embodied without departing from the spirit of the invention.

Next, other characteristic technical matters of the present invention are described below.

### note

(Item 1) An endoscope comprising an operation section in which a trackball can be attached as an instruction section for instructing a predetermined operation; a first engaging section which rotatably engages the trackball with the operation section; a proximal section to which an insertion section that is operated on the basis of an instruction of the operation section is connected; a second engaging section which engages the operation section with the proximal section; and a control member which controls engaging operations of the first engaging section and the second engaging section at the same time.
(Item 2) The endoscope according to item 1, wherein the first engaging section includes a pin member, the proximal section includes a proximal end portion of the insertion section of the endoscope, the second engaging section includes a fixing hook, and the control member includes a rotational lever.
(Item 3) The endoscope according to item 1, wherein the proximal section is attachable/detachable and includes a coupling member having a driving force source for supplying a driving force to the insertion section, and the operation section executes an operational instruction of the driving force source.
(Item 4) The endoscope according to item 3, wherein the proximal section is provided with an engaging section which is engaged with the operation section and guides the operation section in different directions in two steps, a guide direction in a first step is different from a direction of attachment/detachment of the proximal section, and a guide direction in a second step is different from the guide direction in the first step.
(Item 5) The endoscope according to item 4, wherein the engaging section includes first and second slide rails, the guide direction in the first step is a groove direction of the first slide rail, and the guide direction in the second step is a groove direction of the second slide rail.
(Item 6) The endoscope according to item 4, wherein the guide direction in the second step is such a direction as to have resistance against a direction of twisting of the insertion section.
(Item 7) The endoscope according to item 5, wherein the groove direction of the first slide rail and the groove direction of the second slide rail are at right angles with each other.
(Item 8) The endoscope according to item 1, wherein a recess portion for attachment of the trackball is provided in the operation section, and the second engaging section is advancible/retreatable in the recess portion in accordance with the operation of the control member.
(Item 9) The endoscope according to item 1, wherein a first center axis which is a center of grasping at a time of grasping the operation section is eccentric to a second center axis which is a center axis of the insertion section.
(Item 10) A motor-driven bending endoscope including an operation section having operation input sections such as a bending operation input device, an air-feed/water-feed switch, a suction switch and a scope switch, the operation section is being configured to be attachable/detachable to/from a proximal end portion of an insertion section, wherein an attachment/detachment structure between the proximal end portion of the insertion section and the operation section includes a pair of first slide rails and a pair of second slide rails for sliding in a direction different from a direction of the first slide rails, the second slide rails being continuous with the first slide rails at one end, and one of the second slide rails having an abutment portion at the other end, and a hook, which prohibits movement of the operation section relative to the proximal end portion of the insertion section in the state in which the proximal end portion of the insertion section and the operation section abut on each other at the abutment portion, is rotatably provided on the operation section.
(Item 11) The motor-driven bending endoscope according to claim 10, wherein in the operation section which is composed of the hook for fixing the operation section to the proximal end portion of the insertion section, a rotatable operation lever and a pin member which is advancible/retreatable in a trackball mounting recess portion of the operation section in accordance with rotation of the operation lever, the pin member that is advancible/retreatable is coupled to the rotatable operation lever.

### (Industrial Applicability)

The present invention is effective in the technical field of medical apparatuses such as a detachable-insertion-section-type endoscope having a connection section for detachable connection between an insertion section of a motor-driven bending endoscope, a power unit and a conduit connector, and in the technical field in which the medical apparatus is manufactured and used.

## Claims

1. A motor-driven bending endoscope **characterized by** comprising:
an operation section attachment portion (30) which is provided on a proximal end portion of an insertion section (10) including a motor-driven bending section (14); and
an operation section unit (12) which is detachably attached to the operation section attachment portion (30) and includes an operation input section (64) which operates an endoscope (2),
wherein the operation section unit (12) comprises:
a trackball mounting portion (70);
a trackball (45) which is rotatably and attachably/detachably mounted in the trackball mounting portion (70) and is an instruction section for instructing an operation of the bending section (14);
a removal restriction mechanism (72) which restricts removal of the trackball (45), which is mounted in the trackball mounting portion (70), from the trackball mounting portion (70);
an attachment/detachment portion (63) which is detachably coupled to the operation section attachment portion (30) when the operation section unit (12) is attached to the operation section attachment portion (30);
an operation section engaging portion (71) which detachably engages the attachment/detachment portion (63) with the operation section attachment portion (30) when the operation section attachment portion (30) and the attachment/detachment portion (63) are coupled; and
an interlock drive mechanism (73) which is coupled to the operation section engaging portion (71) and the removal restriction mechanism (72) and interlockingly drives the operation section engaging portion (71) and the removal restriction mechanism (72).

2. The motor-driven bending endoscope according to claim 1, **characterized in that** the proximal end portion of the insertion section (10) includes a coupling member (114) which is detachably coupled to a power unit (11) which includes a driving force source for supplying a driving force to the insertion section (10), and
the operation section unit (12) instructs an operation of the driving force source.

3. The motor-driven bending endoscope according to claim 2, **characterized in that** the operation section attachment portion (30) includes an engaging portion (31) for detachably engaging the attachment/detachment portion (63) of the operation section unit (12), and a guide portion (34) for guiding the attachment/detachment portion (63) of the operation section unit (12) to a position of the engaging portion (31),
the guide portion (34) includes guide rails (51a, 51b, 52a, 52b) which guide the operation section unit (12) in two different directions,
a first guide direction of the guide rails (51a, 51b, 52a, 52b) is different from a direction of attachment/detachment of the proximal end portion of the insertion section (10) to/from the power unit (11), and
a second guide direction of the guide rails (51a, 51b, 52a, 52b) is set to be different from the first guide direction.

4. The motor-driven bending endoscope according to claim 3, **characterized in that** the second guide direction is set to be such a direction as to have resistance against a direction of twisting of the insertion section (10) about a center axis of the insertion section (10).

5. The motor-driven bending endoscope according to claim 1, **characterized in that** the operation section unit (12) is provided with the trackball mounting portion (70) on a front surface side of an operation section unit body (61) and is provided with the attachment/detachment portion (63) on a back surface side of the operation section unit body (61),
the trackball mounting portion (70) is formed on the operation section unit (12) and includes a recess portion (69) for attachment of the trackball (45), and
the removal restriction mechanism (72) includes a stopper pin (78) which is advancible/retreatable in the recess portion (69) in accordance with an operation of the interlock drive mechanism (73).

6. The motor-driven bending endoscope according to claim 1, **characterized in that** in the operation section unit (12) a first center axis (O1) which is a center of grasping at a time of grasping is eccentric to a second center axis (02) which is a center axis of the insertion section (10).

7. The motor-driven bending endoscope according to claim 1, **characterized in that** the operation input section (64) includes a bending operation input device (45), an air-feed/water-feed switch (46), a suction switch (47) and a scope switch (48).

8. The motor-driven bending endoscope according to claim 3, **characterized in that** the operation section attachment portion (30) includes first guide rails (51a, 52a) which guide the operation section unit (12) in the first guide direction, and second guide rails (51b, 52b) which guide the operation section unit (12) in the second guide direction, and
the operation section engaging portion (71) is operated at a position where the attachment/detachment portion (63) of the operation section unit (12) is moved to a terminal end position of the second guide rails (51b, 52b).

9. The motor-driven bending endoscope according to claim 1, **characterized in that** the interlock drive mechanism (73) includes a first operation portion (74) which operates the operation section engaging portion (71), a second operation portion (82) which operates the removal restriction mechanism (72), and a rotational lever (65) which rotates about a rotational shaft (76),
the rotational lever (65) includes a lever operation portion (75) at one end side, and includes the first operation portion (74) at the other end side, and
the first operation portion (74) is operated at a terminal end position of a range of rotation of the rotational lever (65), and the second operation portion (82) is operated at an intermediate position of the range of rotation of the rotational lever (65).

10. The motor-driven bending endoscope according to claim 9, **characterized in that** the operation section engaging portion (71) includes a fixing portion (50) which is projectingly provided on the operation section attachment portion (30), and the rotational lever (65) is moved to the terminal end position of the range of rotation when the attachment/detachment portion (63) of the operation section unit (12) is moved to a terminal end position of the second guide rails (51b, 52b), thereby engaging the first operation portion (74) with the fixing portion (50) and restricting movement of the operation section unit (12) along the second guide rails (51b, 52b).

11. A motor-driven bending endoscope **characterized by** comprising:
an operation section attachment portion (30) which is provided on a proximal end portion of an insertion section (10) including a motor-driven bending section (14); and
an operation section unit (12) which is detachably attached to the operation section attachment portion (30) and includes an operation input section (64) which operates an endoscope (2),
wherein the operation section unit (12) comprises:
a trackball mounting portion (70);
a trackball (45) which is rotatably and attachably/detachably mounted in the trackball mounting portion (70) and is an instruction section for instructing an operation of the bending section (14);
removal restriction means (72) for restricting removal of the trackball (45), which is mounted in the trackball mounting portion (70), from the trackball mounting portion (70);
an attachment/detachment portion (63) which is detachably coupled to the operation section attachment portion (30) when the operation section unit (12) is attached to the operation section attachment portion (30);
operation section engaging means (71) for detachably engaging the attachment/detachment portion (63) with the operation section attachment portion (30) when the operation section attachment portion (30) and the attachment/detachment portion (63) are coupled; and
interlock drive means (73) which is coupled to the operation section engaging means (71) and the removal restriction means (72) and interlockingly drives the operation section engaging means (71) and the removal restriction means (72).
